(19) Europäisches Patentamt
European Patent Office
Office européen des brevets

(11) **EP 2 057 206 B1**

(12) **EUROPÄISCHE PATENTSCHRIFT**

(45) Veröffentlichungstag und Bekanntmachung des Hinweises auf die Patenterteilung:
**28.02.2018  Patentblatt 2018/09**

(21) Anmeldenummer: **07802769.5**

(22) Anmeldetag: **21.08.2007**

(51) Int Cl.:
*C07K 14/78* (2006.01)          *C08H 1/06* (2006.01)
*C08L 89/06* (2006.01)

(86) Internationale Anmeldenummer:
**PCT/EP2007/058694**

(87) Internationale Veröffentlichungsnummer:
**WO 2008/023025 (28.02.2008 Gazette 2008/09)**

(54) **HYBRIDMATERIALIEN AUS EINER SILIKATISIERTEN KOLLAGENMATRIX, VERFAHREN ZU DEREN HERSTELLUNG UND DEREN VERWENDUNG**

HYBRID MATERIALS FROM A SILICATE-TREATED COLLAGEN MATRIX, METHODS FOR THE PRODUCTION THEREOF AND THE USE THEREOF

MATÉRIAUX HYBRIDES À MATRICE DE COLLAGÈNE SILICATÉE, PROCÉDÉ POUR LEUR FABRICATION ET UTILISATION DE CES MATÉRIAUX

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **22.08.2006   DE 102006040917**

(43) Veröffentlichungstag der Anmeldung:
**13.05.2009   Patentblatt 2009/20**

(73) Patentinhaber: **Technische Universität Dresden**
**01062 Dresden (DE)**

(72) Erfinder:
• **EHRLICH, Hermann**
  **01728 Gaustritz (DE)**
• **HEINEMANN, Sascha**
  **98574 Schmalkalden (DE)**
• **HANKE, Thomas**
  **13187 Berlin (DE)**
• **WORCH, Hartmut**
  **01187 Dresden (DE)**

(74) Vertreter: **Kailuweit & Uhlemann Patentanwälte Partnerschaft mbB**
**Postfach 32 01 39**
**01013 Dresden (DE)**

(56) Entgegenhaltungen:
**EP-A- 0 415 183     EP-A- 0 540 357**
**MC-A- 200 019**

**EP 2 057 206 B1**

**Beschreibung**

Die Erfindung betrifft neue Hybridmaterialien auf der Basis einer silikatisierten Kollagenmatrix, Verfahren zu deren Herstellung sowie deren Verwendung als vielfältig gestaltbares Implantatmaterial, biologisierende Beschichtung und Wirkstoffträger.

Die Entwicklung neuer Biomaterialien wird hauptsächlich angetrieben durch das Bestreben, ausgereifte Gewebe- und Knochenimplantate für medizinische Anwendungen herzustellen. An diese werden jedoch je nach Implantationsort und Funktion spezielle Ansprüche bezüglich der Biokompatibilität und der mechanischen Festigkeit gestellt. Diese Anforderungen können von einkomponentigen Materialien in der Regel kaum erfüllt werden. Rein biologische Komponenten weisen zwar oftmals eine gute Biokompatibilität auf, sind dabei jedoch nicht in der Lage, größeren mechanischen Belastungen standzuhalten. Aus diesem Grund wird zunehmend an Strategien zur Verstärkung solcher Materialien gearbeitet. Beispielsweise würde dies durch die Kombination mit einer zweiten, lasttragenden Komponente, wie metallischen oder keramischen Werkstoffen erreichbar sein, die ihrerseits wiederum ausreichend biokompatibel sein müssen.

Materialien auf der Basis von Kollagen sind für biomedizinische Anwendungen von besonders großem Interesse. Als körpereigenes Strukturprotein kommt es ubiquitär in allen tierischen Vielzellern vor, und ist mit etwa einem Drittel der gesamten Proteinmasse deren häufigstes Protein. Es ist nahezu untoxisch, bioresorbierbar und kaum immunogen, wodurch sich eine exzellente Biokompatibilität ergibt. Als Ausgangsmaterial für industrielle Anwendungen wird meist Kollagen vom Typ I verwendet, das zum Beispiel aus Sehnen, Knorpel und Häuten von Rindern und Kälbern gewonnen werden kann. Mittlerweile wurden zahlreiche Produkte auf der Basis von Kollagen entwickelt und in vielen Bereichen der Kosmetik und Medizin etabliert. Weitergehende Anwendungen verhindern jedoch oftmals die begrenzten mechanischen Eigenschaften reiner Kollagenprodukte.

Im Tierreich hat die Evolution diesen Mangel eindrucksvoll beseitigt, indem stark belastete Elemente, wie z. B. Knochen aus Materialverbunden aufgebaut werden. So muss Knochen neben anderen Funktionen als Stützelement im Körper vielseitigen mechanischen Belastungen standhalten [Wintermantel, E. ; Ha, S.-W.: Biokompatible Werkstoffe und Bauweisen. Springer Verlag, 1998]. Trotz einer hohen Festigkeit (Druckspannungen) muss er jedoch auch eine gewisse Elastizität (Zug- und Biegespannungen) aufweisen, damit er auch unter ungünstigen Belastungszuständen (Hebelkräfte) nicht spröde bricht. Diesen Anforderungen wird der Knochen durch seinen hierarchischen Aufbau und ganz entscheidend auch durch seine stoffliche Zusammensetzung auf molekularer Ebene gerecht. Die organische Knochenmatrix, die ca. 30% des Knochens ausmacht, besteht zu ca. 95% aus Kollagen, das den Part der elastischen Komponente zur Aufnahme von Zug- und Biegespannungen einnimmt. Im Zuge eines Mineralisationsprozesses wird zwischen den Kollagenfibrillen mineralisches Calciumphosphat (vor allem Hydroxylapatit) in Gestalt etwa zwei bis vier Nanometer dicker kristalliner Plättchen eingelagert [Kapitel Forschung aktuell. In:MaxPlankForschung (Hrsg.): Knochen auf den Zahn gefühlt. Bd. 1. 2005, S. 10-1]. Je nach Grad der Mineralisation (beim Knochen ca. 65 %) wird das flexible Grundgerüst aus Kollagen mechanisch verstärkt, wodurch sich die Festigkeit, vor allem unter Druckbelastung, enorm erhöht.

Aufbauend auf derartigen Erkenntnissen wird seit einiger Zeit intensiv an Möglichkeiten zur Nachahmung natürlich vorkommender Verbundmaterialien - vor allem des Knochens - gearbeitet, wobei jedoch einige Beschränkungen einzuhalten sind. Die direkte Synthese solcher Verbundwerkstoffe scheiterte bisher an den Hochtemperaturverfahren, in denen metallische und keramische Werkstoffe hergestellt werden, da die Temperaturbeständigkeit der Biokomponenten meist auf deutlich niedrigere Bereiche beschränkt ist. Besonderes Interesse liegt darum bei Verfahren, die eine Synthese bei Raumtemperatur zulassen. So wurden Fällungsverfahren entwickelt, mit Hilfe derer Kollagen unter Ausbildung von Calciumphosphatphasen mineralisiert werden kann. Weitere Entwicklungen zeigten, dass sich sogenanntes Tropokollagen bereits während dessen Fibrillogenese mit Hydroxylapatit mineralisieren lässt und sich aus diesem Kollagen hergestellte Scaffolds durch verbesserte mechanische Eigenschaften auszeichnen [Gelinsky, M. ; König, U. ; Sewing, A. ; Pompe, W.: Poröse Scaffolds aus mineralisiertem Kollagen - ein biomimetisches Knochenersatzmaterial. In: Materialwiss Werkstofftech 35 (2004), Nr. 4, S. 229-33].

Eine weitere interessante Alternative bietet der bei Raumtemperatur durchführbare Sol-Gel-Prozess. Dieser ermöglicht es, ausgehend von einer flüssigen Lösung - einem Sol - anorganisch-nichtmetallische Partikel zu synthetisieren, die unter bestimmten Bedingungen bis zur Ausbildung eines stabilen dreidimensionalen Netzwerkes - eines Gels - agglomerieren können. Als besonders aussichtsreich für die Übertragung in biologische Systeme auf diese Weise gilt amorphes Siliziumdioxid - Glas. Auf analoge Weise werden heute bioaktive Keramiken synthetisiert, die u. a. als Knochenersatzmaterial eingesetzt werden sollen. Die mechanischen Eigenschaften unterscheiden sich vom natürlichen Knochen jedoch durch höhere Sprödigkeit und geringere Elastizität.

Durch die Kombination flexibler organischer Polymere mit Grundbestandteilen der bioaktiven Keramiken konnten organisch-anorganische Hybridmaterialien hergestellt werden, deren mechanische Eigenschaften denen des natürlichen Knochenmaterials sehr nahe kamen [Miyazaki, T. ; Ohtsuki, C. ; Tanihara, M.: Synthesis of bioactive organicinorganic nanohybrid for bone repair through sol-gel processing. In: J Nanosci Nanotechnol 3 (2003), Nr. 6, S. 511-5. -

1533-4880]. Ausgehend von den genannten Vorteilen erhofft man sich darüber hinaus, dass die physikalische Einlagerung von Biokomponenten in Sol-Gel-Gläser neue Möglichkeiten für die Biotechnologie eröffnet. Erste Berichte über die Einlagerung von Enzymen wurden bereits in den frühen 70er Jahren veröffentlicht. Seit dieser Zeit berichten zahlreiche Publikationen über den Einbau von Proteinen, darunter Enzyme und Antikörper sowie Bakterien und sogar Zellen in Sol-Gel-Gläser [Nassif, N. ; Bouvet, O. ; Noelle Rager, M. ; Roux, C. ; Coradin, T. ; Livage, J.: Living bacteria in silica gels. In: Nat Mater 1 (2002), Nr. 1, S. 42-4. - 1476-1122]. Alle Ergebnisse zeigten, dass die eingelagerten Komponenten ihre biologische Aktivität beibehielten [Livage, J. ; Coradin, T. ; Roux, C.: Encapsulation of biomolecules in silica gels. In: J Phys Condens Matter 190 (2001), S. 673-91. - 0953-8984].

Beispiele für eine organische Matrix, die mit einer anorganisch nichtmetallischen Mineralphase assoziiert ist, finden sich bei den 600 Millionen Jahre alten marinen Glasschwämmen [Ehrlich, H. ; Hanke, T. ; Simon, P. ; Goebel, C. ; Heinemann, S. ; Born, R.; Worch, H.: Demineralization of natural silica based biomaterials: new strategy for the isolation of organic frameworks. In: BIOmaterialien 6 (2005), Nr. 4, S. 297-302] [Ehrlich, H. ; Ereskovskii, A. V. ; Drozdov, A. L. ; Krylova, D. D. ; Hanke, T. Meissner, H. ; Heinemann, S. ; Worch, H.: A Modern Approach to Demineralization of Spicules in Glass Sponges (Porifera: Hexactinellida) fort he Purpose of Extraction and Examination of the Protein Matrix. In: Russ Marin Biol 32 (2006), Nr. 3, S. 186-93]. Am Beispiel der Glasnadeln (Spikulen) des Glasschwammes Hyalonema sieboldi konnte mithilfe eines neuartigen alkalischen Demineralisationsverfahrens die Mineralphase - amorphes Siliziumdioxid (biogener Opal) - gelöst und dadurch die organische Phase weitgehend ungeschädigt freigelegt werden. Eine anschließende Isolierung und Identifizierung derselben ergab verblüffende Übereinstimmungen mit bekannten Kollagenen. Es kann vermutet werden, dass dieses Kollagen, äquivalent zu den beim Knochen bekannten Bildungsmechanismen, als biologisches Templat für die Mineralisation dient.

Durch Untersuchungen mittels Röntgenabsorptionsspektroskopie (NEXAFS - Near Edge X-ray Absorption Fine Structure) konnte ermittelt werden, dass ca. 64 Masse-% einer solchen Glasnadel aus Kollagen bestehen. Das als flexibles Gerüst dienende Kollagen ist in den Spikulen dieser Schwämme vollständig mit Siliziumdioxid mineralisiert und schichtet sich in ca. 20 Nanometer dicken Lagen um eine zentrale Achse (Axialfilament). Aufgrund dieses hohen Kollagenanteils und der hierarchischen Struktur ergibt sich für die Spikulen eine erstaunliche Flexibilität, so dass sie sogar zu einem Knoten gebunden werden können [Levi, C. ; Barton, J. L. ; Guillemet, c. ; Le Bras, E. ; Lehuede, P.: A remarkably strong natural glassy rod: the anchoring spicule of the Monorhaphis sponge. In: J Mater Sci Lett 3 (1989), S. 337-9]. Erste Untersuchungen haben gezeigt, dass Kollagenfibrillen als formgebende Schablone für die Herstellung anorganischer Strukturen genutzt werden können. Dazu wurden einzelne Fibrillen des Kollagens Typ I gleichmäßig mit einer Silikatschicht überzogen. Anschließend wurden die organischen Bestandteile (also das Kollagen) vollständig zerstört und Hohlfasern aus Silikat erhalten [Ono, Y. ; Kanekiyo, Y. ; Inoue, K. ; Hojo, J. ; Nango, M. ; Shinkai, S.: Preparation of Novel Hollow Fiber Silica Using Collagen Fibers as a Template. In: Chem Lett (1999), S. 475-6]. Auch wurde in Ansätzen gezeigt, dass sich das Vorhandensein von Kieselsäure bereits während der Fibrillogenese auf die Morphologie der resultierenden Fibrillen auswirkt [Eglin, D. ; Coradin, T. ; Giraud Guille, M. M. ; Helary, C. ; Livage, J.: Collagen-silica hybrid materials: sodium silicate and sodium chloride effects on type I collagen fibrillogenesis. In: Biomed Mater Eng 15 (2005), Nr. 1-2, S. 43-50. - 0959-2989]. Außerdem beschrieben Coradin et al., 2002 [Coradin et al., A novel route to collagen-silica biohybrids, in: Organic/Inorganic Hybrid Materials, C. Sanchez, R.M. Laine, S.Yang and C.J. Brinker, eds, Materials Research Society Symposium Proceedings 726: 79-83 (2002)] und Eglin et al., 2004 [Eglin et al., Collagen-silica hybrid materials: Sodium silicate and sodium chloride effects on type I collagen fibrillogenesis, Bio-Medical Materials and Engineering 15:43-49 (2005)] Kollagen-Siliziumdioxid-Verbünde, die auf Kollagengel-Matritzen beruhten. Sie wurden aus Rattenschwanz-Sehnen-Kollagen und Natriumsilikat durch Ko-Gelierung hergestellt. Sie stellten fest, dass die Zusammensetzung Einfluss auf die gebildeten Strukturen hat. Die Experimente beschränkten sich jedoch auf enge Konzentrationsbereiche beider Komponenten und die Herstellung mikroskopischer Proben für Strukturuntersuchungen.

Die EP 0 415 183 A2 offenbart vernetzte Hydrogele zur Anwendung für offene Wunden, Blut oder für den dauerhaften Verbleib als Implantat im menschlichen Körper und ein Verfahren zu deren Herstellung. Die Hydrogele bestehen beispielsweise aus löslichem Kollagen und einer Alkoxysilylverbindung als Vernetzer. Die eingesetzte Alkoxysilylverbindung, wie beispielsweise Tetramethoxysilan oder Tetraethoxysilan, liegt dabei im Hydrogel in ihrer ursprünglichen Struktur kovalent gebunden an das Kollagen vor. Unter löslichem Kollagen sind einzelne Kollagenmoleküle zu verstehen, die als Tripelhelices vorliegen und im Hydrogel einen Anteil von 40 - 95 Gew.-% haben.

Weiterhin offenbart die EP 0 450 357 A2 Copolymere aus beispielsweise Kollagen und einem Silikon. Copolymere sind Makromoleküle aus verschiedenen monomeren Bausteinen (Kollagen und Silikon), wobei diese kovalent verbunden sind. Die eingesetzten Kollagene weisen Molmassen im Bereich von 500 bis 500.000 Dalton auf und sind wasserlöslich. Die Silikone besitzen funktionelle Gruppen, über diese sie fest mit dem Kollagen kovalent verbunden werden. Die ursprüngliche monomere Struktur der Silikone bleibt dabei im Copolymer erhalten.

Die MC 200 019 A offenbart Komplexe aus beispielsweise Kollagen und Tetraalkoxysilanen. Besonders gut zur Komplexbildung geeignet ist hydrolysiertes Kollagen. Hydrolysiertes Kollagen wird durch Erwärmen und/oder Säure-/Basenbehandlung von Kollagen erhalten. Es ist das Denaturierungsprodukt aus der genannten Umsetzung. Zur Bildung der Komplexe wird schließlich ein Siliziumprecusor, wie beispielsweise Tetraalkoxysilan eingesetzt. Dieser wird durch Hydrolyse zu Orthokieselsäure umgesetzt. Das hydrolysierte Kollagen dient schließlich als Stabilisator für die Orthokieselsäure und verhindert, dass diese zu Siliziumdioxid(-partikeln) kondensiert.

Alle genannten Erkenntnisse beschränken sich jedoch auf ausgewählte Grundlagenexperimente. Praktisch nutzbare, makroskopische Hybridmaterialien aus Kollagen und biomimetisch erzeugtem Silikat sind aus dem Stand der Technik bislang nicht bekannt.

Aufgabe der Erfindung ist es daher, neue Hybridmaterialien zum Einsatz in der Medizin und Biotechnologie bereitzustellen, die biokompatibel sind und auch größeren mechanischen Belastungen standhalten können. Insbesondere sollten die Hybridmaterialien neben einer hohen Festigkeit (Druckspannung) auch eine gewisse Elastizität (Zug- und Biegespannung) aufweisen, damit sie auch unter ungünstigen Belastungszuständen (Hebelkräfte) nicht spröde brechen.

Erfindungsgemäß wird die Aufgabe gelöst durch ein Hybridmaterial bestehend aus einer silikatisierten Kollagenmatrix, wobei das Kollagen rekombinantes Kollagen, Kollagen aus Eumetazoa (d.h. Gewebetiere, darunter Nesseltiere und Bilateria), Schwammkollagen der Klassen Demospongia (Hornschwämme) oder Calcarea (Kalkschwämme), ein synthetisches Kollagenanalogon, ein Kollagenderivat oder eine Mischung dieser Kollagene ist und wobei die Kollagenmatrix durch die Silikatisierung der Kollagenfibrillen versteift und vernetzt ist.

Kollagenanaloga sind synthetisch hergestellte Polypeptidketten, deren Primärsequenz so gestaltet ist, dass sie bestimmte Eigenschaften der nativen Kollagenmonomere simulieren können, z.B. Tripelhelixbildung, Fibrillogenese, Gelbildung.

Rekombinante Kollagene sind solche, deren Primärsequenz identisch ist mit der von Kollagen Typ I. Sie werden mithilfe genetisch manipulierter Mikroorganismen hergestellt und gegebenenfalls nachbehandelt.

Kollagenderivate sind Verbindungen, die sich von der Grundverbindung Kollagen formal ableiten und aus ihr herstellen lassen.

Das erfindungsgemäße Hybridmaterial ist erhältlich durch Mischen einer homogenen Kollagensuspension und eines Silizium-Precursors.

Bestandteil der Erfindung ist auch ein Verfahren zur Herstellung eines Hybridmaterials aus einer silikatisierten Kollagenmatrix, wobei als Kollagen rekombinantes Kollagen, Kollagen aus Eumetazoa, Schwammkollagen aus einem Schwamm der Klasse Demospongia (Hornschwämme) oder Calcarea (Kalkschwämme), ein synthetisches Kollagenanalogon, ein Kollagenderivat oder eine Mischung dieser Kollagene verwendet wird, wobei die Kollagenmatrix durch die Silikatisierung versteift und vernetzt wird indem eine homogene Kollagensuspension und ein Silizium-Precursor unter Rühren vermischt werden.

Die Erfindung beruht auf der überraschenden wissenschaftlichen Erkenntnis, dass in den Basalspikulen des marinen Glasschwamms Hyalonema sieboldi Kollagen als Hauptkomponente vorliegt und dessen Silikatisierung wahrscheinlich zu den ungewöhnlichen mechanischen Eigenschaften der Spikulen führt [Ehrlich, H. ; Hanke, T. ; Simon, P. ; Goebel, C. ; Heinemann, S. ; Born, R. ; Worch, H.: Demineralization of natural silica based biomaterials: new strategy for the isolation of organic frameworks. In: BIOmaterialien 6 (2005), Nr. 4, S. 297-302].

Der zugrundeliegende Silikatisierungsprozess von Schwamm-Kollagen kann überraschenderweise synthetisch nachgestellt werden. Dazu wurde unter gezielt gewählten Randbedingungen (pH 7,4 und 20°C) im Labor ein Alkoxysilan zu Kieselsäure hydrolysiert und diese in definierten Konzentrationen mit homogenen Suspensionen von Kollagen versetzt. Mittels quantitativer Methoden konnte nachgewiesen werden, dass die Kieselsäurepolymere mit den Aminogruppen des Kollagens reagieren. Zeitaufgelöste Messungen haben außerdem ergeben, dass das Kollagen konzentrationsabhängig die heterogene Keimbildung der Kieselsäurepolymerisation an den Fibrillen selbst deutlich beschleunigt.

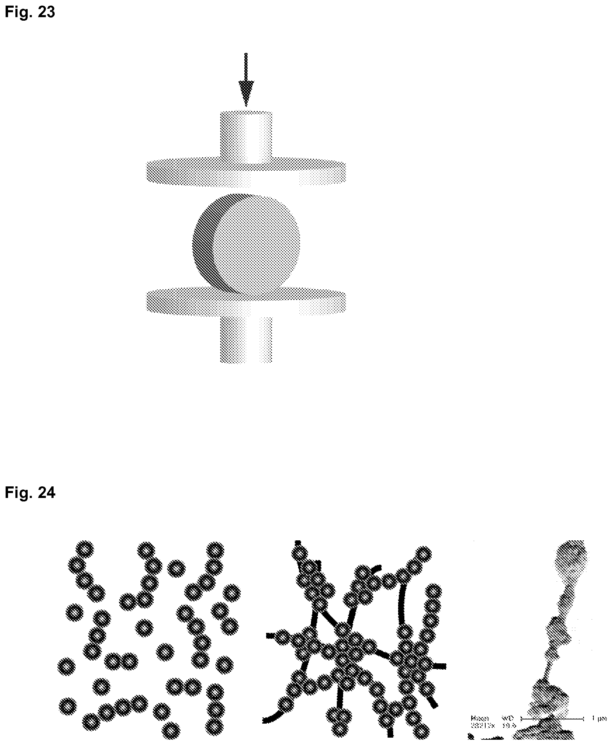

Anhand atomkraft- und rasterelektronenmikroskopischer Aufnahmen konnte gezeigt werden, dass sich unter den experimentellen Bedingungen nanometergroße Silikatpartikel an den Fibrillen des Kollagens bilden, agglomerieren und diese somit silikatisieren (Fig. 24).

Die im Rahmen des Sol-Gel-Prozesses gebildete Kieselsäure vernetzt bei der Polykondensation zu Silikat einzelne Kollagenmoleküle oder -fibrillen miteinander oder untereinander. Außerdem verstärkt die im Rahmen des Sol-Gel-Prozesses gebildete Kieselsäure bei der Polykondensation zu Silikat ein bereits bestehendes Kollagennetzwerk.

Dieser Silikatisierungsprozess wurde anschließend auf die Herstellung makroskopischer Hybridmaterialien aus einer silikatisierten Kollagenmatrix übertragen.

Das erfindungsgemäße Hybridmaterial aus einer silikatisierten Kollagenmatrix ist biokompatibel und hält auch größeren mechanischen Belastungen stand. Insbesondere weisen die Hybridmaterialien neben einer hohen Festigkeit (Druckspannung) auch eine gewisse Elastizität (Zug- und Biegespannung) auf, so dass sie auch unter ungünstigen Belastungszuständen (Hebelkräfte) nicht spröde brechen.

Das erfindungsgemäße Hybridmaterial aus einer silikatisierten Kollagenmatrix ist außerdem strukturell und stofflich in weiten Grenzen modifizierbar und somit zur Herstellung vielfältigster Applikationen mit mechanisch und biologisch angepassten Eigenschaften einsetzbar. Durch die Nutzung der Sol-Gel-Technik kann das Material in verschiedenen geometrischen Formen hergestellt werden.

Als Kollagenkomponente des erfindungsgemäßen Hybridmaterials kann jegliches Kollagen eingesetzt werden, d.h. Kollagen jeglicher Form (z.B. fibrilläres Kollagen, netzbildendes Kollagen, faserbildendes Kollagen, Tropokollagen (einzelne Kollagenmoleküle), teilweise oder vollständig fibrilliertes Kollagen, teilweise oder vollständig (irreversibel) denaturiertes Kollagen, Kollagenfasern oder größere Aggregate usw.) und jeglichen Typs (z.B. Typ I, II, III, IV, V, VI, VII, VIII, IX, X, XI, XII, XIII, XIV, XV, XVI, XVII, XVIII, XIX, XX, XXI, XXII, XXIII, XXIV, XXV, XXVI).

In einer bevorzugten Ausführungsform des Hybridmaterials ist das Kollagen ein Wirbeltierkollagen, vorzugsweise Kalbshautkollagen oder Schweinekollagen, oder Kollagen aus dem marinen Hornschwamm Chondrosia reniformis.

Wirbeltierkollagen weist dabei vorteilhaft eine hohe Biokompatibilität auf. Ferner ist es nicht toxisch und nicht immunogen.

Bei Verwendung von Schwammkollagen können Infektionen durch pathogene Keime, Prionen etc. ausgeschlossen werden. Besonders vorteilhaft erweist sich dabei der hohe Kollagenanteil des marinen Hornschwamms Chondrosia reniformis. Außerdem ist dieser Schwamm essbar, was auf eine geringe Allgemeintoxizität des Schwammes selbst und auch des daraus gewonnenen Kollagens schließen lässt.

Entsprechend kann beim erfindungsgemäßen Verfahren zur Herstellung des Hybridmaterials als Kollagenkomponente jegliches Kollagen eingesetzt werden, d.h. Kollagen jeglicher Form (z.B. fibrilläres Kollagen, netzbildendes Kollagen, faserbildendes Kollagen, Tropokollagen (einzelne Kollagenmoleküle), teilweise oder vollständig fibrilliertes Kollagen, teilweise oder vollständig (irreversibel) denaturiertes Kollagen, Kollagenfasern oder größere Aggregate usw.) und jeglichen Typs (z.B. Typ I, II, III, IV, V, VI, VII, VIII, IX, X, XI, XII, XIII, XIV, XV, XVI, XVII, XVIII, XIX, XX, XXI, XXII, XXIII, XXIV, XXV, XXVI).

In einer bevorzugten Ausführungsform des erfindungsgemäßen Verfahrens zur Herstellung des Hybridmaterials wird als Kollagen Kalbshautkollagen, Schweinekollagen oder Kollagen aus dem marinen Hornschwamm Chondrosia reniformis eingesetzt.

Verfahren zur Gewinnung von Kollagen sind dem Fachmann bekannt.

Schwammkollagen wird nach dem Fachmann bekannten Verfahren isoliert und gereinigt und liegt im Wesentlichen frei von Verunreinigungen beispielsweise durch andere Proteine vor. Vorschriften zur Isolierung von Schwammkollagen sind beispielsweise publiziert in Jungua et al., Biochemical and morphological studies on the collagen of horny sponges. Ircinia filaments compared to spongines, Connective Tissue Research 2: 193-203 (1974); Swatschek et al., Marine sponge collagen: isolation, characterization and effects on the skin parameters surface-pH, moisture and sebum, European Journal of Pharmaceutics and Biopharmaceutics 53: 107-113 (2002)). Nach EP1259120 A ist Schwammkollagen aus dem Hornschwamm Chondrosia reniformis beispielsweise erhältlich, indem in Ethanol konserviertes Material des Hornschwamms Chondrosia reniformis dreimal unter fließendem Wasser gewaschen, in kleine Teile geschnitten und homogenisiert wird, während ein Tris-HCl-Puffer-Gemisch (100 mM, pH 9,5; 10 nM EDTA; 8 M Harnstoff; 10 mM 2-Mercaptoethanol) hinzugefügt wird. Nach ständigem Rühren bei Raumtemperatur über 24 Stunden hinweg wird der viskose Extrakt zentrifugiert. Das Pellet wird verworfen und Kollagen wird aus dem Überstand durch Einstellung des pH auf 4,0 mittels Essigsäure ausgefällt und durch Zentrifugation letztlich gewonnen.

Um die für alle Silikatisierungsreaktionen vorausgesetzte Kieselsäure zu synthetisieren, wird erfindungsgemäß ein Silizium-Precursor eingesetzt. In einer bevorzugten Ausführungsform des Hybridmaterials ist der Silizium-Precursor eine hydrolysierte Alkoxysilan-Lösung (Si(OR)$_4$, R=C$_n$H$_{2n+1}$) oder Wasserglas (Na$_2$SiO$_3$). In einer besonders bevorzugten Ausführungsform ist die Alkoxysilan-Lösung eine Tetramethoxysilan (TMOS (Si(OCH$_3$)$_4$) - Lösung oder eine Tetraethoxysilan (TEOS) - Lösung.

Im Verfahrens zur Herstellung des erfindungsgemäßen Hybridmaterials wird erfindungsgemäß als Silizium-Precursor eine hydrolysierte Alkoxysilan-Lösung oder Wasserglas (Na$_2$SiO$_3$) eingesetzt. In einer besonders bevorzugten Ausführungsform wird als Alkoxysilan-Lösung eine Tetramethoxysilan (TMOS) - Lösung oder eine Tetraethoxysilan (TEOS) - Lösung eingesetzt.

Durch die Hydrolyse werden die Alkoxy-Gruppen der Alkoxysilan-Lösung - meist unter Katalyse mit Salzsäure (HCl) - durch Wasser hydrolisiert und dabei zunehmend durch OH-Gruppen ersetzt.

$$Si(OR)_n + n\ H_2O \rightarrow Si(OH)_n + n\ R\text{-}OH$$

Bei dieser Reaktion entstehen Orthokieselsäure und ein vom Silizium-Precusor abhängiger Alkohol. Im Falle des Tetramethoxysilans (TMOS) entspricht z.B. R = CH$_3$ und n = 4, so dass bei der exothermen Hydrolyse Methanol entsteht. Die gebildeten Silanol-Guppen (Si-OH) reagieren im zweiten Schritt - der Polykondensation - miteinander oder mit noch unhydrolysierten Alkoxy-Gruppen des Silans unter Ausbildung von Siloxanbindungen (Si-O-Si).

$$Si\text{-}OH + HO\text{-}Si \rightarrow Si\text{-}O\text{-}Si + H_2O$$

$$Si\text{-}OR + HO\text{-}Si \rightarrow Si\text{-}O\text{-}Si + R\text{-}OH$$

Die Gesamtreaktion kann somit geschrieben werden als

$$Si(OR)_4 + 2\ H_2O \rightarrow SiO_2 + 4\ R\text{-}OH.$$

Dabei werden die Alkoxygruppen des Silans nach und nach durch OH-Gruppen substituiert:

Monomere Kieselsäuremoleküle kondensieren in geringem Maße selbstständig, aber vor allem unter dem katalytischen Einfluss der Templatfunktion der Kollagen-Mikrofibrillen zu Nanopartikeln und bilden amorphes Siliziumdioxid.

Kondensation

- Monomer + Monomer → Dimer + Wasser
- $Si(OH)_4 + HO\text{-}Si(OH)_3 \rightarrow (OH)_3Si\text{-}O\text{-}Si(OH)_3 + H_2O$

Bildung von Siliziumdioxid-Nanopartikeln

- Dimere → Trimer → Tetramer →... → $SiO_2$-Partikel

Das in der Lösung vorliegende Kollagen dient als biologisches Templat und erzwingt die gezielte Polymerisation der Kieselsäure bzw. des Silikats an den Kollagenfibrillen. Die Keimbildung beruht auf den attraktiven Wechselwirkungen zwischen den positiv geladenen Aminogruppen des Kollagens und den negativ geladenen Oxidgruppen der Kieselsäure bzw. Silikatpartikel.

In Abhängigkeit von Kollagen- und eingesetzter Alkoxysilan-Konzentration wird eine Silikatisierung der Kollagenfibrillen erzielt, was bei weiterer Entwicklung zur Ausbildung kompakter Hybridmaterialien führt, bei denen die Kollagenmatrix durch die Silikatisierung versteift und vernetzt ist.

Derartige Hybridmaterialien sind biomedizinisch als Implantatmaterial einsetzbar, bei dem neben guter Biokompatibilität eine hinreichende Festigkeit (z. B. im Knochenkontakt) gefordert wird.

Das Masseverhältnis von Kollagen zu Silikat lässt sich in einem weiten Zusammensetzungsbereich variieren. Erfindungsgemäß ist der Zusammensetzungsbereich von etwa 30 bis 70 Masse-% Kollagen.

Als Lösungsmittel der Kollagensuspension können prinzipiell alle Lösungsmittel eingesetzt werden, die das Kollagen nicht in seinen notwendigen Eigenschaften verändern und außerdem bei Zusammenführung mit der Silikatkomponente die genannten Mineralisationsprozesse erlauben. Gleiches gilt für die Lösung des Silikat-Precursors.
Der pH-Wert der Ausgangslösungen bzw. der Mischung ist vorzugsweise im neutralen oder annähernd neutralen Bereich und wird vorzugsweise nahe den physiologischen Bedingungen (um pH 7,4) gewählt. Abänderungen zu höheren bzw. niedrigeren pH-Werten können genutzt werden, um z. B. die Reaktionskinetik zu beeinflussen.

Ausgehend vom erfindungsgemäßen Hybridmaterial sind verschiedene Materialien herstellbar:

- Ausgehend von homogenen Kollagensuspensionen und geringen Zusätzen an Kieselsäure sind durch Gefriertrocknung silikatisierte poröse Kollagenscaffolds herstellbar.

- Ausgehend von homogenen Kollagensuspensionen und einem entsprechend gesteigerten Anteil an Kieselsäure wird der Sol-Gel-Prozess ermöglicht, wobei die Silikatphase zu einem stabilen dreidimensionalen Netzwerk (Gel) reagiert. Auf diese Weise sind Hydrogele herstellbar.

- Die Lufttrocknung von Hydrogelen führt zu entsprechenden Xerogelen.

- Die Kritisch-Punkt-Trocknung der Hydrogele führt zu Aerogelen.

- Hybridbeschichtungen und Hybridpartikel sind ebenfalls herstellbar.

Anhand der zuvor dargelegten Erkenntnisse kann eine auf die gewünschten Hybridmaterialien und die dazu verwendeten Verfahren jeweils zugeschnittene Abgrenzung geeigneter Zusammensetzungsbereiche bezüglich des Kollagens und der Alkoxysilan-Lösung vorgenommen werden.

Somit ist es möglich, die mechanischen Eigenschaften der erfindungsgemäßen Hybridmaterialien auf Basis von Kollagen und Silikat in einem weiten Bereich für technische und biomedizinische Anwendungen gezielt anzupassen.

Bestandteil der Erfindung ist auch ein silikatisiertes poröses Kollagenscaffold, das durch Gefriertrocknung eines erfindungsgemäßen Hybridmaterials erhältlich ist.

Bestandteil der Erfindung ist auch ein Verfahren, bei dem zur Herstellung eines silikatisierten porösen Kollagenscaffolds eine homogene Kollagensuspension und geringe Mengen einer hydrolysierten Alkoxysilan-Lösung gemischt werden und anschließend eine Gefriertrocknung durchgeführt wird.

Für die Herstellung von silikatisierten porösen Kollagenscaffolds wird eine homogene Kollagensuspension hergestellt. Je nach verwendetem Kollagen kann die Konzentration dabei so weit gesteigert werden, wie sich die erhaltene Suspension noch homogen mit der Silikat-Komponente vermischen lässt. Diese wird mit einer pre-hydrolysierten Alkoxysilan-Lösung geeigneter Konzentration vermischt.

Um den Charakter eines porösen Kollagenscaffolds - und nicht den eines Silikatgels - zu erhalten, darf die Alkoxysilan-Konzentration bzw. deren Verhältnis zur eingesetzten Kollagenkonzentration (typischerweise ca. 40 mg/ml) kollagentyp-abhängige Obergrenzen nicht überschreiten. Die einsetzbaren Konzentrationen sind stark von der Natur des Kollagens und weiteren Reaktionsbedingungen abhängig. Mit steigender Kollagenkonzentration ist eine Abnahme der nach Gefriertrocknung erhaltenen mittleren Porengröße verbunden. Außerdem nimmt die Reaktionsgeschwindigkeit der Kieselsäurepolymerisation zu. Änderungen des pH-Werts wirken sich wie oben genannt ebenfalls auf die Reaktionsgeschwindigkeit aus.

Nach Überführung in eine geeignete Form wird eine Gefriertrocknung durchgeführt. In direktem Zusammenhang mit den Komponentenkonzentrationen steht die Reaktionsdauer, während der die Kieselsäurepolymerisation bzw. die Silikatisierung stattfindet, bevor die entsprechenden Prozesse durch Gefriertrocknung abgebrochen werden.

Die Silikatisierung durch Zugabe von bis zu 100 mM TMOS bei 40 mg/ml Kollagen, z.B. Chondrosia-Kollagen oder Kalbshautkollagen, führt z.B. zu keinen wesentlichen Veränderungen der makroskopisch sichtbaren Strukturen gegenüber nicht-silikatisierten reinen Kollagenscaffolds. Im Gegensatz dazu führt eine Erhöhung des Silikatanteils durch TMOS-Konzentrationen von 0,250 M und mehr innerhalb der gesetzten Reaktionszeit zur Gelbildung. Die nach der Gefriertrocknung erhaltenen Scaffolds zeigten dabei strukturelle Defekte in Form von Rissen, die möglicherweise durch die Schädigung des Silikatnetzwerks während des Einfrier- und Trocknungsvorgangs verursacht wurden. Hochauflösende rasterelektronenmikroskopische Untersuchungen zeigen, dass bei den silikatisierten Scaffolds Silikatpartikel in die Kollagenmatrix eingelagert wurden und diese somit auch mechanisch stützen.

Unter Beibehaltung der makroskopischen und mikroskopischen Architektur der Kollagen-Scaffolds führt die Silikatisierung des Scaffolds zu einer Erhöhung des Elastizitätsmoduls gegenüber silikatfreien Kollagen-Scaffolds. Silikatisierte Kollagen-Scaffolds weisen also vorteilhaft gegenüber nicht silikatisierten Kollagen-Scaffolds deutlich erhöhte Elastizitätsmodulen auf. Mit steigendem Silikatisierungsgrad erhöhen sich die Elastizitätsmodulen entsprechender Scaffolds stetig. So weist z.B. ein silikatisierter Kollagen-Scaffold, der aus 50 mM TMOS und 40 mg/ml Kollagen erhalten wurde, einen Elastizitätsmodul von etwa 0,76 MPa auf, ein silikatisierter Kollagen-Scaffold, der aus 75 mM TMOS und 40 mg/ml Kollagen erhalten wurde, weist einen Elastizitätsmodul von etwa 1,25 MPa auf. Bei einer TMOS-Konzentration von 100 mM (40 mg/ml Kollagen) wurden mittlere Elastizitätsmodulen von etwa 1,48 MPa ermittelt. Ein nichtsilikatisierter Kollagen-Scaffold, der aus einer Kollagenlösung von 40 mg/ml erhalten wird, weist demgegenüber einen Elastizitätsmodul von nur etwa 0,40 MPa auf.

Als poröser Scaffold erfüllt das erfindungsgemäße Hybridmaterial alle Kriterien für erfolgreiches Tissue Engineering. Bestandteil der Erfindung ist daher auch die Verwendung des silikatisierten porösen Kollagenscaffolds zum Tissue Engineering.

Ebenfalls Bestandteil der Erfindung ist ein Hydrogel, das durch Lagern eines erfindungsgemäßen Hybridmaterials unter Luftabschluss nach Erreichen des Gelpunkts zur Festigung der Festkörperstruktur in einem geeigneten Lösungsmittel erhältlich ist. Bestandteil der Erfindung ist ebenfalls ein Verfahren bei dem zur Herstellung eines Hydrogels eine homogene Kollagensuspension und eine hydrolysierte Alkoxysilan-Lösung gemischt werden und das Produkt nach Erreichen des Gelpunkts zur Festigung der Festkörperstruktur in einem geeigneten Lösungsmittel unter Luftabschluss gelagert wird.

Dabei wird auch bei niedrigen Temperaturen durch das Mischen einer homogenen Kollagensuspension und einer hydrolysierten Alkoxysilan-Lösung (Kieselsäure) der Sol-Gel-Prozess ermöglicht. Die kontinuierliche Zusammenlagerung von Silizium über die Ausbildung der Siloxan-Bindungen führt zur Bildung von Polymeren bzw. Partikeln. Sind diese noch separiert im Lösungsmittel vorhanden, spricht man von einem Sol, also einer kolloidalen Lösung. In Abhängigkeit von der Partikelkonzentration und deren Größe führt die fortschreitende Kondensation der Silikatpartikel zur Erhöhung der Viskosität. Sobald die Kondensation zur Ausbildung eines zusammenhängenden dreidimensionalen SilikatNetzwerks führt - diese Zeitmarke wird als Gelpunkt bezeichnet - erhält man ein stabiles Silikat-Gel. Das Kollagen dient in diesem Fall als organisches Templat und wird während der Herstellung des Gels in dieses eingelagert.

Solange der Gelpunkt noch nicht erreicht ist, lässt sich die Lösung in nahezu beliebige Formen bringen und sich somit die endgültige Probengestalt bestimmen. Eine Festigung der Festkörperstruktur (Alterung) wird durch Tränken der Hydrogele in einem geeigneten Lösungsmittel und Lagerung unter Luftabschluss erreicht. Dabei laufen die Prozesse der so genannten Synerese und der Ostwald-Reifung ab.

Der mögliche Zusammensetzungsbereich der Gele, Reaktionsbedingungen sowie die einsetzbaren Konzentrationen der beiden Ausgangskomponenten sind von wiederum von zahlreichen, sich gegenseitig bedingenden Parametern abhängig. Besonders geeignet ist ein Zusammensetzungsbereich von 0 - 100 mg / ml Kollagen (pH 7,4 in 0,1M Tris/HCl-Puffer) bei 0,25 - 1,00 M TMOS (hydrolysiert in $H_2O$/HCl).

Die erfindungsgemäßen Hydrogele lassen sich - abhängig vom angestrebten Anwendungsfall - prinzipiell folgendermaßen weiterverwenden:

- Weiterverwendung als Hydrogel

- Trocknung an Luft, was zur Bildung eines Xerogels führt

- Trocknung unter überkritischen Bedingungen, was zur Bildung eines Aerogels führt

Ein weiterer Bestandteil der Erfindung ist ein Xerogel, das durch Trocknen, vorzugsweise Lufttrocknen eines erfindungsgemäßen Hydrogels erhältlich ist.

Bestandteil der Erfindung ist auch ein Verfahren, bei dem zur Herstellung eines Xerogels ein erfindungsgemäßes Hydrogel getrocknet, vorzugsweise luftgetrocknet wird.

Das Xerogel wird erhalten durch Entfernung der flüssigen Phase (zum Teil Wasser bzw. Alkohol) aus dem Hydrogel. Je nach Festphasenanteil kann die Schrumpfung dabei bis zu 90% bei einem verbleibenden Porenvolumen von bis zu 50% betragen. Die dabei entstehenden Kapillarkräfte können zu Trocknungsrissen bis hin zur völligen Zerstörung des Festkörpers mit dem Ergebnis eines Pulvers führen. Eine vorgeschaltete Substitution durch zum Beispiel Alkohole kann die Beanspruchung der Festphase bei Trocknen verringern. Das Trocknungsregime mit seinen Parametern wie Temperatur und Luftfeuchtigkeit hat einen entscheidenden Einfluss auf die Erhaltung der Festkörperstruktur. So werden bei sehr langsamer Trocknung in einem Klimaschrank monolithische Xerogele erhalten.

Die Herstellung von Xerogelen geht von den erfindungsgemäßen Hydrogelen aus. Der Zusammensetzungsbereich von Alkoxysilan- und Kollagenlösung sowie die Reaktionsbedingungen entsprechen denen der Hydrogele. Die Kollagenkonzentrationen beeinflusst in Kombination mit entsprechenden Alkoxysilan-Konzentrationen das Trocknungsverhalten der Hydrogele und damit die Eigenschaften der resultierenden Xerogele. Im Gegensatz zu reinen Silikat-Hydrogelen werden Hydrogele aus dem erfindungsgemäßen Hybridmaterial vorzugsweise langsam, d.h. ca. zwei bis drei Tage, bei Raumklima luftgetrocknet.

Hydrogele aus Kollagen und Silikat weisen gegenüber Hydrogelen aus reinem Silikat eine erhöhte Spaltzugfestigkeit und eine erhöhte Bruchdehnung aus. Durch das Vorhandensein des eingelagerten Kollagens wird eine deutliche Verbesserung der elastischen Eigenschaften der Xerogele erzielt.

Ein Masseanteil von 30 bis 70 % Kollagen ist besonders bevorzugt. Dabei werden die höchsten gemessenen Spaltzugfestigkeiten und Bruchdehnungen erreicht. Eine weitere Erhöhung der Kollagenkonzentration ruft nur geringe zusätzliche Kennwertänderungen der Xerogele hervor. Bei Xerogelen mit höherem Kollagenanteil ist sogar wieder ein deutlicher Rückgang der Spaltzugfestigkeit und Bruchdehnung festzustellen. Dieser Abfall lässt sich möglicherweise mit dem Überschreiten eines als optimal einzustufenden Masseverhältnisses von Silikat zu Kollagen erklären.

Xerogele aus Kollagen und Silikat, insbesondere Xerogele auf Basis von Chondrosia-Kollagen bzw. Kalbshaut-Kollagen Typ I und Silikat, sind vorteilhaft bei Zellkulturtests gut haltbar und handhabbar. Sie lassen sich aufgrund des geringen Wassergehalts problemlos mit Gamma-Strahlung sterilisieren, ihre hohe spezifische Dichte verhindert ein Aufschwimmen im Zellkulturmedium, was in Verbindung mit der ebenen Probenoberfläche entsprechende Zellversuche deutlich vereinfacht. Darüberhinaus ermöglicht die einheitliche Probengeometrie sogar quantitative Proliferationsmessungen.

Insbesondere auf Basis von Kalbshautkollagen Typ I und Silikat hergestellte Xerogele lassen sich sehr gut für zellbiologische Untersuchungen verwenden. Mittels quantitativer und qualitativer Methoden konnte die Proliferation und gute Anpassung von Mausosteoblasten an die Oberfläche derartiger Materialien nachgewiesen werden.

Bestandteil der Erfindung ist auch ein Aerogel, das durch Substitution der Flüssigphase eines erfindungsgemäßen Hydrogels und anschließende Trocknung unter überkritischen Bedingungen erhältlich ist.

Bestandteil der Erfindung ist auch ein Verfahren zur Herstellung eines Aerogels, bei dem die Flüssigphase eines erfindungsgemäßen Hydrogels substituiert und im Anschluss eine Trocknung unter überkritischen Bedingungen durchgeführt wird.

Die Herstellung von Aerogelen geht von Hydrogelen aus, d. h. es gelten gleiche Bedingungen an Ausgangslösungen und Reaktionsbedingungen.

Im Falle der Aerogele wird die Flüssigphase so aus dem Hydrogel entfernt, dass keine (bzw. nur sehr geringe) Kapillarkräfte auftreten und somit eine Schrumpfung des Materials vermieden wird. Die anzuwendende Trocknung unter überkritischen Bedingungen erfordert zunächst eine Substitution der Flüssigphase (z.B. Wasser und Methanol bei Verwendung von TMOS als Silikat-Precursor) der Hydrogele. Dazu werden die Hydrogele z.B. mehrere Tage in Reinstethanol eingelegt. Das Ethanol wird dabei regelmäßig in Abständen gewechselt. Danach wird die Kritisch-Punkt-Trocknung in einem Autoklaven durchgeführt. Dabei wird das Ethanol zunächst bei einer Temperatur von etwa 7°C unter Hochdruckbedingungen durch mehrmaliges Spülen durch flüssiges Kohlendioxid ersetzt. Anschließend wird die Temperatur auf 38 bis 40°C erhöht, wobei das Kohlendioxid in den überkritischen Zustand übergeht, d. h. es existiert keine Grenzfläche mehr zwischen der flüssigen und gasförmigen Phase. Durch ein Ventil wird das überkritische Kohlendioxid bei Aufrechterhaltung der Bedingungen langsam abgeblasen. Durch das Vermeiden der Grenzfläche können keine Kapillarkräfte entstehen und die Festkörperstruktur der Probe bleibt unverändert erhalten. Nach Abschluss dieses Verfahrens werden getrocknete Aerogele erhalten, deren innere und äußere Strukturen denen der eingesetzten Hydrogele entsprechen.

Bestandteil der Erfindung ist die Verwendung eines erfindungsgemäßen Hydrogels, Xerogels oder Aerogels als Zahnfüllung, als Implantatmaterial, als Knochenersatzmaterial und für die Fixierung von Knochenbrüchen (Osteosynthese) und für die Knochenregeneration.

In Form kompakter Gele (d. h. als Hydrogel, Xerogel oder Aerogel) können die erfindungsgemäßen Hybridmaterialien aufgrund ihrer Biokompatibilität und ihren mechanischen Eigenschaften z. B. als Zahnfüllung, Implantatmaterial und für die Fixierung von Knochenbrüchen (Osteosynthese) und die Knochenregeneration zum Einsatz kommen.

Insbesondere erfindungsgemäße Hybrid-Xerogele, z.B. aus Kalbshautkollagen und Silikat, eignen sich zur Verwendung als Knochenersatzmaterial, d. h. mit Hilfe des Hybridmaterials können z.B. Knochendefekte ausgefüllt oder auch ganze Knochen ersetzt werden.

Die besondere Eignung des Hybrid-Xerogels, z.B. aus Kalbshautkollagen und Silikat für die Verwendung als Knochenersatzmaterial resultiert aus der besonderen Eigenschaftskombination des erfindungsgemäßen Hybrid-Xerogels, insbesondere aus dessen guten und variabel einstellbaren mechanischen und zellbiologischen Eigenschaften: Auf dem erfindungsgemäßen Hybrid-Xerogel kultivierte humane mesenchymale Stammzellen reagieren besonders positiv, proliferieren und differenzieren in Knochenzellen. Vorteilhaft können die erfindungsgemäßen Hybrid-Xerogele durch das Herstellungsverfahren gleich in die gewünschte Zielform (Platten, Kugeln, Stifte) gebracht werden. Um spezielle Geometrien zu erhalten, erfolgt eine mechanische Bearbeitung (z. B. spanabhebend) der Probekörper. Alternativ wird das Hybrid-Material vor seiner Erstarrung in einen (Knochen)defekt eingebracht und härtet dort aus.

Bestandteil der Erfindung sind auch Partikel aus dem erfindungsgemäßen Hybridmaterial. Diese sind erhältlich, indem eine homogene Kollagensuspension zu einer Alkoxysilan-Lösung gegeben wird.

Bestandteil der Erfindung ist auch ein Verfahren, bei dem zur Herstellung von Partikeln eine homogene Kollagensuspension zu einer Alkoxysilan-Lösung gegeben wird.

Zur Herstellung sphärischer Partikel werden entsprechend konzentrierte homogene Kollagensuspensionen tropfenweise in eine reine Alkoxysilan-Lösung pipettiert. Dabei bildet sich sofort eine Kugel aus, die nach ca. 24 Stunden aus der Lösung genommen, gespült und luftgetrocknet wird. Das resultierende Produkt entspricht somit einem sphärisch geformten Hybrid-Xerogel.

Beim Eintropfen von Kollagensuspensionen (bei Verwendung von Schwammkollagen beispielsweise bis ca. 100 mg/ml) neutralen pH-Werts (z. B. Tris/HCl-Puffer) in eine 98%ige TMOS-Lösung führt eine lokale Hydrolyse durch das Wasserangebot und die Templatfunktion des Kollagens zur spontanen Polymerisation der Kieselsäure mit dem Ergebnis eines kugelförmigen Silikat-Kollagen-Hybridpartikels.

Als Partikel ist das erfindungsgemäße Hybridmaterial z. B. als Wirkstoffträger ("Drug delivery system") geeignet. Bestandteil der Erfindung ist daher auch die Verwendung des Partikels als Wirkstoffträger.

Bestandteil der Erfindung ist auch ein Beschichtungsmaterial aus einem erfindungsgemäßen Hybridmaterial sowie ein Verfahren, bei dem zur Beschichtung eines Substrats mit einem Hybridmaterial eine Kollagensuspension und eine Alkoxysilan-Lösung gemischt werden, das Substrat in die Mischlösung getaucht wird und das Substrat anschließend getrocknet wird. Bestandteil der Erfindung ist auch die Verwendung dieser Beschichtung zur Biologisierung von Implantatoberflächen.

Die Konzentrationen der Kollagensuspension und der Alkoxysilan-Lösung werden dabei so niedrig gewählt, dass die Mischlösung den Gel-Punkt im Versuchszeitraum nicht erreicht. Zur Beschichtung werden die Substrate (z. B. biokompatible metallische Platten) in die Mischlösung getaucht und langsam herausgezogen. Dabei scheidet sich eine Schicht auf den Substraten ab, deren Dicke und Beschaffenheit vor allem von der Zusammensetzung der Tauchlösung und der Ziehgeschwindigkeit abhängen. An Luft trocknen die Schichten zu einem dichten und mechanisch stabilen Substratüberzug.

Bestandteil der Erfindung ist die Verwendung des erfindungsgemäßen Hybridmaterials als Konstruktionswerkstoff und / oder als Funktionswerkstoff sowie als elektronisches Bauelement oder als Implantatmaterial.

Die erfindungsgemäßen Hybridmaterialien können prinzipiell für technische und biologische Zwecke genutzt werden. Auf technischer Seite ist der Einsatz in nano- und mikroelektronischen Bauelementen sowie die Nutzung der optischen Eigenschaften des Materials möglich. Bei biologischer und insbesondere bei medizinischer Anwendung kann das neuartige Hybridmaterial Beschränkungen aktuell verfügbarer Implantatmaterialien überwinden.

Bestandteil der Erfindung ist auch ein Knochenersatzmaterial, das erfindungsgemäßes Hybridmaterial enthält.

Anhand nachfolgender Figuren und Ausführungsbeispiele wird die Erfindung näher erläutert, ohne diese einzuschränken.

Dabei zeigen

**Fig. 1** Durch Gefriertrocknung erhaltene poröse Scaffolds (Durchmesser jeweils ca. 15 mm) auf Basis von Chondrosia-Kollagen (40 mg/ml). Die äußerlich sichtbare Gestalt ändert sich vom reinen Kollagenscaffold (a) mit steigender TMOS-Konzentration (b: 0,025 M, c: 0,050 M, d: 0,075 M, e: 0,100 M TMOS) zunächst nur geringfügig. Bei einer TMOS-Konzentration von 0,250 M ist die Struktur bereits merklich dichter und der Gel-Charakter tritt hervor (f).

**Fig. 2** Von der TMOS- und Kollagenkonzentration abhängiges Anwendungsspektrum. Die schraffierten Felder grenzen die als geeignet erkannten Zusammensetzungsbereiche für die Herstellung von porösen Scaffolds (S), Aerogelen (A), Hydrogelen (H), Xerogelen (X), Partikeln (P), und Beschichtungen (B) ein.

**Fig.3** Dreidimensionale Rekonstruktion der gemessenen µCT-Daten eines silikatisierten porösen Chondrosia-Kollagenscaffolds. Der Durchmesser des zylindrischen Probenausschnitts beträgt ca. 4 mm.

**Fig. 4** REM-Aufnahmen der porösen Scaffolds auf Basis des Chondrosia-Kollagens. Die Teilbilder auf der linken Seite (a, c, e) zeigen einen reinen Kollagenscaffold (40 mg/ml), die rechten Teilbilder (b, d, f) einen mit 0,1 M TMOS silikatisierten Scaffold gleicher Kollagenkonzentration.

**Fig. 5** Anhand mechanischer Testungen berechnete Elastizitätsmoduln der reinen und silikatisierten (25 - 100 mM TMOS) porösen Scaffolds auf Basis des Chondrosia-Kollagens.

**Fig. 6** Durch Gefriertrocknung nach verschiedenen Trocknungs- bzw. Alterungszeiten (a: 1 h, b: 1 d, c: 2 d) und nach Lufttrocknung (d: 3 d) erhaltene Silikat-Xerogele (1,00 M TMOS). Durchmesser der Vertiefungen: 16,2 mm.

**Fig. 7** Durch Gefriertrocknung nach verschiedenen Trocknungs- bzw. Alterungszeiten (a: 1 h, b: 1 d, c: 2 d) und nach Lufttrocknung (d: 3 d) erhaltene Hybrid-Xerogele (0,50 M TMOS, 50 mg/ml Chondrosia-Kollagen).

**Fig. 8** REM-Aufnahmen der reinen Silikat- und Hybrid-Xerogele. Die Teilbilder auf der linken Seite (a, c, e) zeigen ein reines Silikat-Xerogel (1,0 M TMOS), die rechten Teilbilder (b, d, f) ein Hybrid-Xerogel (1,0 M TMOS, 50 mg/ml Chondrosia-Kollagen).

**Fig. 9** FEM-Simulation des Versuchs zur Bestimmung der Spaltzugfestigkeit der Xerogele. Im Teilbild (a) ist die räumliche Verteilung der Spannung in y-Richtung, im Teilbild (b) die der Spannung in x-Richtung qualitativ dargestellt.

**Fig. 10** Bruchverhalten der reinen Silikat-Xerogele (a) und Hybrid-Xerogele mit Chondrosia-Kollagen (b) beim Druckversuch zur Bestimmung der Spaltzugfestigkeit. Linke Proben jeweils vor, rechte Proben nach der Testung (Probendurchmesser ca. 6 mm).

**Fig. 11** Anhand des Druckversuchs zur Bestimmung der Spaltzugfestigkeit ermittelte elastische Eigenschaften der reinen Silikat- und Hybrid-Xerogele bei Variation der TMOS- und Kollagenkonzentration.

**Fig. 12** Aufnahmen der für die Zellbesiedlung genutzen Xerogele: reines Silikat (a), Hybrid mit Chondrosia-Kollagen (b) und Hybrid mit Kalbshaut-Kollagen Typ I (c). Die Durchmesser der Proben belaufen sich jeweils auf etwa 5 mm.

**Fig. 13** Fluoreszenz- (a) und LSM-Aufnahme (b) der sieben Tage auf dem reinen Silikat-Xerogel kultivierten Mausosteoblasten 7F2. Dargestellt sind die Zellkerne und die Aktingerüste der Zellen.

**Fig. 14** MTT-Test: Auf den Tag 1 bezogene Enzymaktivitäten bei Kultivierung von Mausosteoblasten 7F2 auf den Hybrid-Xerogelen und Vergleichsmaterialien.

**Fig. 15** Fluoreszenz-Aufnahmen der auf den mit Kalbshautkollagen Typ I beschichteten Polystyrol-Wellplatten kultivierten Zellen 24 Stunden (a, c) und sieben Tage (b, d) nach der Besiedlung. Dargestellt sind die Zellkerne und die Aktingerüste der Zellen.

**Fig. 16** LSM-Aufnahmen der auf den Hybrid-Xerogelen kultivierten Mausosteoblasten 24 Stunden (a, c) und sieben Tage (b, d) nach der Besiedlung. Dargestellt sind die Zellkerne und die Aktingerüste der Zellen.

**Fig. 17** REM-Aufnahmen der auf den Hybrid-Xerogelen kultivierten Maus-ostoblasten sieben Tage nach der Besiedlung.

**Fig. 18** Hergestellte Silikat- und Hybrid-Aerogele mit einer Höhe von etwa 8 mm bei 15 mm Durchmesser. Das in Teilbild (a) gezeigte reine Silikat-Aerogel entspricht einer TMOS-Konzentration von 1,00 M. In Teilbild (b) ist ein Hybrid-Aerogel mit 50 mg/ml Chondrosia-Kollagen bei 0,50 M TMOS abgebildet.

**Fig. 19** REM-Aufnahmen eines Hybrid-Aerogels zusammengesetzt aus 0,5 M TMOS und 50 mg/ml Chondrosia-Kollagen.

**Fig. 20** Nach Lufttrocknung erhaltene reine Silikatpartikel (a) beziehungsweise unter Einsatz von Chondrosia-Kollagensuspensionen (50 mg/ml) erhaltene Hybridpartikel (b).

**Fig. 21** REM-Aufnahmen eines Hybrid-Partikels bestehend aus silikatisiertem Chondrosia-Kollagen

**Fig. 22** Finite-Elemente-Modell mit Kennzeichnung der Randbedingungen und des Lastfalls zur Simulation des Versuchs zur Bestimmung der Spaltzugfestigkeit der Xerogele

**Fig. 23:** Schematische Darstellung der Versuchsanordnung zur Bestimmung der Spaltzugfestigkeit der Xerogele

**Fig. 24:** Durch Auto-Polykondensation der Silikatpartikel (dunkle Punkte) bildet sich ein dreidimensionales Gelnetzwerk. Durch die Anwesenheit eines organischen Templats kann der Silikatisierungsprozess gezielt gesteuert werden, wie es z. B. für die Verwendung von (fibrillärem) Kollagen gezeigt wurde (mittleres Schema, invertiertes REM-Bild im rechten Teilbild.

**Fig. 25:** Relative Masse der nicht-entwässerten und entwässerten Hydrogele während des Trocknungsvorganges. Werte über 100% resultieren von zugeführter Flüssigkeit während des Alterungsschrittes.

**Fig. 26:** Fotografie der scheibenförmigen, monolithischen (Durchmesser = 5mm, Höhe = 2 mm) Silikat-Kollagen-Hybridxerogele (70 % $SiO_2$, 30 % Kollagen) wie sie im optimierten Herstellungsprozess erhalten wurden.

**Fig. 27:** Laser-Scanning-Mikroskopiebild von humanen mesenchymalen Stammzellen, nach 24-stündiger Kultivierung auf den Silikat-Kollagen-Hybridxerogelen (links). Nach 14 Tagen bilden sich dichte Zellagglomerate infolge der Proliferation. In derselben Region zeigt sich eine intensive Aktivität der alkalischen Phosphatase infolge osteogener Differenzierung.

**Fig. 28:** DNA-Gehalt bei der Kultivierung von humanen mesenchymalen Stammzellen auf Silikat-Kollagen-Hybridxerogelen bzw. auf Polystyrol. Die osteogene Differenzierung wurde am Tag 3 eingeleitet.

**Fig. 29:** Auf den DNA-Gehalt bezogene ALP-Aktivität bei der Kultivierung von humanen mesenchymalen Stammzellen auf Silikat-Kollagen-Hybridxerogelen bzw. auf Polystyrol. Die osteogene Differenzierung wurde am Tag 3 eingeleitet.

**Fig. 30:** REM-Aufnahmen der Silikat-Kollagen-Hybridxerogele nach eintägiger (a, b) und 14-tägiger (c, d) Kultivierung von osteogen induzierten humanen mesenchymalen Stammzellen.

**Ausführungsbeispiel 1**

**Herstellung von porösen Kollagenscaffolds und silikatisierten porösen Kollagenscaffolds**

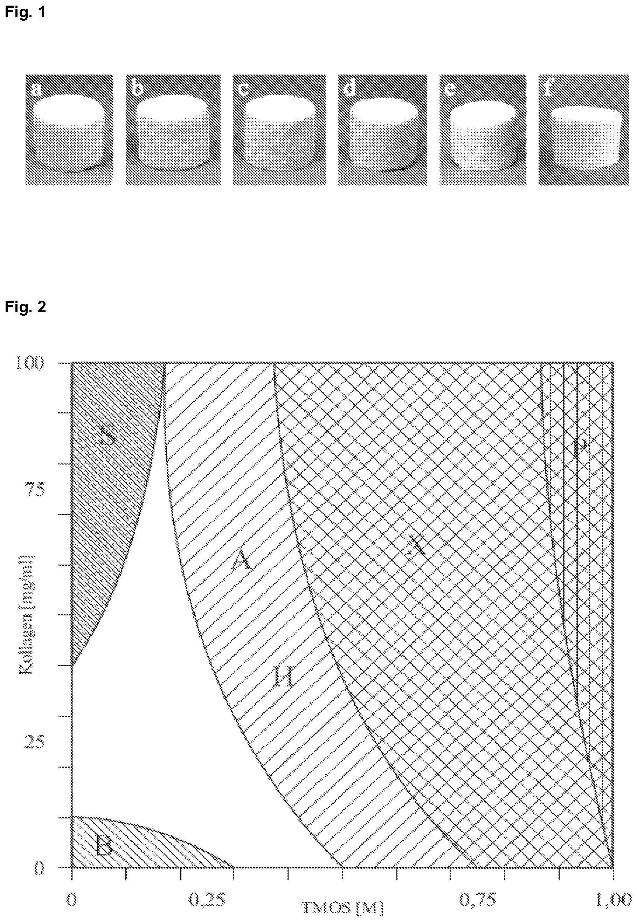

Für die Herstellung reiner poröser Kollagenscaffolds wurde zuvor aufbereitetes Chondrosia-Kollagenlyophilisat in 0,1 M Tris/HCl-Puffer (pH 7,4) bzw. PBS-Lösung (pH 7,4) in einer Konzentration von 40 mg/ml über einen Zeitraum von 48 Stunden bei 4°C zu einer homogenen Suspension verrührt. Anschließend wurden jeweils 2,5 ml dieser Suspensionen in die Vertiefungen von 24er Wellplatten pipettiert und bei -80°C eingefroren. Danach wurde eine Gefriertrocknung durchgeführt. Die erhaltenen Scaffolds wurden bei 4°C im Exsikkator aufbewahrt. Auf diese Weise wurden einheitliche Kollagenscaffolds definierter Größe (Durchmesser ca. 15 mm, Höhe ca. 10mm) erhalten. Deren Gestalt zeigt das Teilbild (a) in **Fig. 1.** Die durchschnittliche Masse und mittlere Dichte dieser Scaffolds ist in Tabelle 1 zusammengefasst.

**Tab. 1:**

| Masse und mittlere Dichte der reinen und silikatisierten porösen Scaffolds in Abhängigkeit von der TMOS-Konzentration beim Einsatz von 40 mg/ml Chondrosia-Kollagen (Gesamtvolumen jeweils 2,5 ml). | | | | | | |
|---|---|---|---|---|---|---|
| **TMOS-Konzentration [M]** | **0,000** | **0,025** | **0,050** | **0,075** | **0,100** | **0,250** |
| **Berechnete Masse [mg]** | 100,00 | 103,68 | 107,37 | 111,05 | 114,73 | 136,83 |
| **Gemessene Masse [mg]** | 101,02 ±2,97 | 103,20 ±1,61 | 109,57 ±3,06 | 112,38 ±2,84 | 116,65 ±2,61 | 135,65 ±2,27 |
| **Scheinbare Dichte [mg/cm$^3$]** | 49,45 ±0,62 | 54,40 ±0,93 | 58,47 ±0,70 | 61,13 ±1,16 | 62,60 ±0,53 | 82,03 ±3,17 |

Für die Herstellung von silikatisierten porösen Kollagenscaffolds wurden zu Beginn entsprechend höher konzentrierte homogene Kollagensuspensionen hergestellt. Diese wurden in jeweils separaten Zentrifugenröhrchen mit pre-hydrolysierten TMOS-Lösungen geeigneter Konzentrationen auf dem Vortexer vermischt, so dass letztendlich die genannte Kollagenkonzentration und TMOS-Konzentrationen von 25, 50, 75, 100 bzw. 250 mM erhalten wurden. Die Reaktionszeit wurde auf 30 Minuten begrenzt. Der Inhalt von ebenfalls 2,5 ml wurde schließlich direkt in die Vertiefungen von 24er Wellplatten gegossen, um Masseverluste und Blasenbildung zu vermeiden. Die weitere Verfahrensweise war identisch zu den reinen Kollagenscaffolds. Die nach Einfrieren und Gefriertrocknung erhaltenen silikatisierten Kollagenscaffolds sind in den Teilbildern (b - e) der Fig. 1 zu sehen. Die nach der Gefriertrocknung ermittelten Massen und mittleren Dichten der porösen Scaffolds sind in Tabelle 1 ersichtlich.

Wie der Tabelle zu entnehmen ist, steigt die Gesamtmasse und auch die mittlere Dichte der Scaffolds mit zunehmendem Silikatanteil. Die gute Übereinstimmung der gemessenen mit den berechneten Massen weist darauf hin, dass das in hydrolysierter Form eingesetzte TMOS (Kieselsäure) vollständig zu Silikat umgesetzt und in die Scaffolds eingebaut wurde. Die Versuche ergaben außerdem, dass die unter den genannten Bedingungen hergestellten Hybridmaterialien bis zu einer TMOS-Konzentration von etwa 0,100 M den Charakter der reinen Kollagenscaffolds behielten. Im Gegensatz dazu, führte eine Erhöhung des Silikatanteils durch TMOS-Konzentrationen von 0,250 M und mehr, innerhalb der gesetzten Reaktionszeit zur Gelbildung. Die nach der Gefriertrocknung erhaltenen Scaffolds zeigten dabei strukturelle Defekte in Form von Rissen, die möglicherweise durch die Schädigung des Silikatnetzwerks während des Einfrierund Trocknungsvorgangs verursacht wurden. Neben den aufgeführten Beispielen konnten silikatisierte Kollagenscaffolds geringerer Dichte durch gleichzeitige Verringerung der Kollagen- und TMOS-Konzentration erhalten werden. Der Zusammensetzungsbereich für die Herstellung der wie gewünscht homogen silikatisierten Kollagenscaffolds, konnte somit entsprechend eingegrenzt werden (siehe **Fig. 2**).

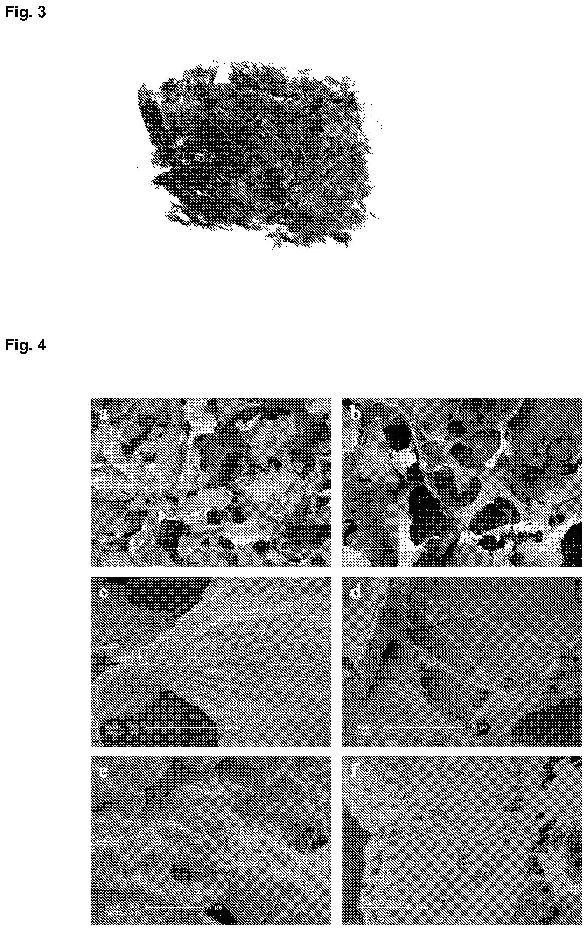

Durch die Untersuchung der hergestellten reinen und silikatisierten porösen Chondrosia-Kollagenscaffolds mittels Mikro-Computertomografie (μCT) und Rasterelektronenmikroskopie (s. Ausführungsbeispiel 6) wurde die Verteilung des Silikats auf makroskopischer, mikroskopischer und nanoskopischer Ebene untersucht. Mittels μCT wurde exemplarisch ein mit 0,1 M TMOS silikatisierter poröser Chondrosia-Kollagenscaffold (40 mg/ml) analysiert und anhand der Daten eine dreidimensionale Rekonstruktion erstellt, die in **Fig. 3** zu sehen ist. Aufgrund der elementspezifischen Absorption der Röntgenstrahlen können die abgebildeten, grün erscheinenden Strukturen vollständig der starken Absorption des Silikats zugeordnet werden. Reines Kollagen würde aufgrund seiner nur schwachen Absorption kein Signal ergeben. Anhand der Grafik kann festgestellt werden, dass die Verteilung des Silikats scheinbar der Struktur der organischen Kollagenmatrix folgt - das Silikat demzufolge kein eigenständiges Netzwerk aufbaut. Die Verteilung ist zudem recht homogen, was auf eine gute Durchmischung der Komponenten bei der Herstellung und eine gleichmäßige Polymerisation des TMOS im gesamten Volumen schließen lässt.

Eine nähere Charakterisierung der Architektur wurde nach entsprechender Präparation mittels Rasterelektronenmikroskopie durchgeführt. Einige exemplarische Aufnahmen dieser Untersuchungen sind in **Fig. 4** zusammengestellt. Hier stehen sich jeweils REM-Aufnahmen reiner Chondrosia-Kollagenscaffolds (40 mg/ml) auf der linken Seite (a, c, e) und die eines mit 0,1M TMOS silikatisierten Kollagenscaffolds auf der rechten Seite (b, d, f) in abgestimmten Vergrößerungen gegenüber. Bei kleineren Vergrößerungen, wie sie in den Teilbildern (a) und (b) zu sehen sind, wird die großporige Gestalt interkonnektierender Hohlräume deutlich. Die mittleren Porengrößen betragen ca. 100 - 150 μm, was für die zelluläre Besiedlung häufig als Idealmaß angesehen wird [Sachlos, E. ; Czernuszka, J. T.: Making tissue engineering scaffolds work. Review: the application of solid freeform fabrication technology to the production of tissue engineering scaffolds. In: Eur Cell Mater 5 (2003), S. 29-39; discussion 39-40. - 1473-2262 (Electronic) Journal Article Review]. Auffällige strukturelle Unterschiede zwischen beiden Scaffoldtypen sind an dieser Stelle noch nicht festzustellen, so dass keine Aussagen zum Einfluss der Silikatisierung getroffen werden können. Die in den Teilbildern (c) und (d) dargestellten Aufnahmen geben dagegen schon tiefere Einblicke in die mikroskopische Organisation der Komponenten. Beim reinen Chondrosia- Kollagenscaffold wird die Substruktur der Porenwände in Form einer gerichteten Rauhigkeit sichtbar. Beim silikatisierten Scaffold hingegen erscheinen diese Oberflächen glatter. An einigen Stellen sind einzelne Kollagenfibrillen und Fibrillenbündel erkennbar, an denen Silikat in Form einzelner Partikel und größerer Aggregate angelagert ist. Bei den in den Teilbildern (e) und (f) dargestellten REM-Aufnahmen höherer Vergrößerung sind die Unterschiede im makromolekularen Aufbau zwischen beiden Proben eindeutig zu erkennen. Die Wände des reinen Kollagenscaffolds zeichnen sich durch eine geschlossene und wellige Oberfläche aus. Der Aufbau und die Zusammensetzung aus Kollagen kann an einigen Stellen anhand frei liegender Fibrillen erkannt werden. Einen ganz anderen Aufbau zeigen die REM-Aufnahmen des silikatisierten Kollagenscaffolds. Hier wird bei ausreichender Vergrößerung der enge Verbund aus maschenartig aufgespannten Kollagenfibrillen sichtbar, die fast vollständig mit Silikatpartikeln belegt sind. Die gegenseitige Vernetzung und Überbrückung von Leerräumen durch das Silikat führt zur Ausbildung dieses insgesamt homogen erscheinenden Materialverbundes. Dieser deutlich unterschiedliche mikroskopische Aufbau der reinen Scaffolds aus Chondrosia- Kollagen bzw. dem Hybridmaterial lässt erwarten, dass der Einbau des Silikats entsprechende Auswirkungen auf die mechanischen Eigenschaften hat.

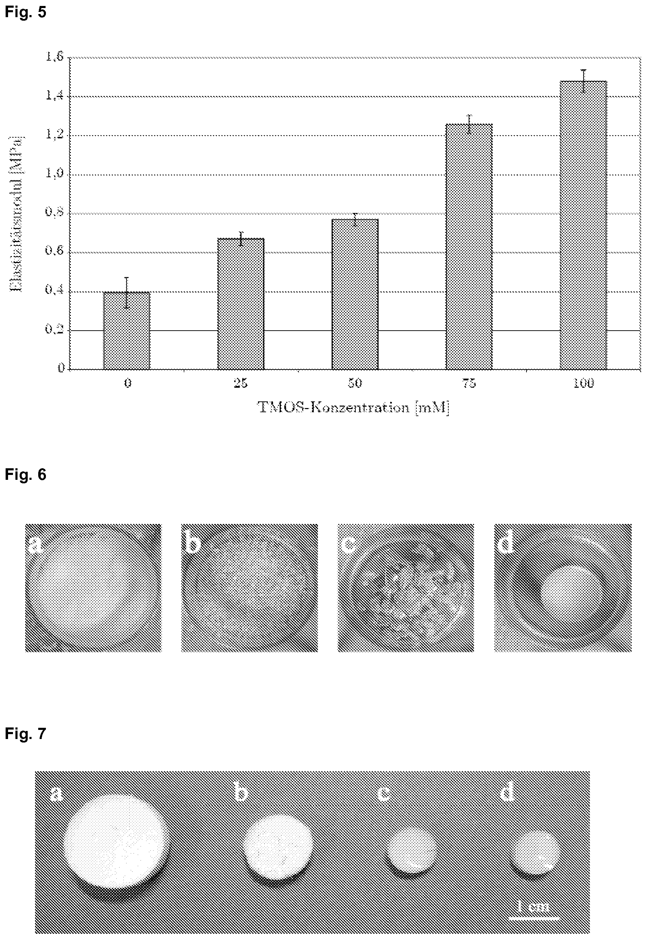

Mit dem Ziel, die dadurch möglicherweise hervorgerufenen Änderungen der elastischen Eigenschaften der Scaffolds zu untersuchen, wurden die in Abschnitt 6.3 von Ausführungsbeispiel 6 beschriebenen mechanischen Testungen durchgeführt. Anhand der Messergebnisse wurden die jeweiligen Elastizitätsmodulen berechnet und diese in Abhängigkeit von der TMOS-Konzentration in **Fig. 5** dargestellt. Wie dem Diagramm zu entnehmen ist, liegt der Elastizitästmodul der reinen Chondrosia-Kollagenscaffolds mit einer Konzentration von 40mg/ml Kollagen bei etwa 0,40 MPa. Bereits die Zugabe von 25 mM TMOS während des Herstellungsprozesses und die damit verbundene Silikatisierung der Fibrillen des Chondrosia-Kollagens äußert sich in einer deutlichen Erhöhung des Elastizitätsmoduls auf etwa 0,65 MPa. Die Steigerung der TMOS-Konzentration und damit verbundene Zunahme des Silikatisierungsgrads wirkt sich in einer stetigen Erhöhung der Elastizitätsmoduln entsprechender Scaffolds aus. So steigen diese bei 50 mM TMOS auf etwa 0,76 MPa und bei 75 mM TMOS auf etwa 1,25 MPa. Bei der in dieser Versuchsreihe höchsten TMOS-Konzentration (100mM) wurden mittlere Elastizitätsmodulen von etwa 1,48 MPa ermittelt. Bei Beibehaltung der makroskopischen und mikroskopischen Architektur der Scaffolds führt die unter diesen Bedingungen erreichte Silikatisierung demzufolge zu einer Erhöhung des Elastizitätsmoduls um etwa den Faktor 3,75 gegenüber den silikatfreien Vegleichsproben.

**Ausführungsbeispiel 2**

**Silikat- und Kollagen-Silikat-Hybrid-Hydrogele**

Die Herstellung von Silikat- und Hybrid-Hydrogelen erfolgte in zwei Schritten.
Zunächst wurde eine Pre-Hydrolyse der TMOS-Lösung durchgeführt, um die für alle Silikatisierungsreaktionen vorausgesetzte Kieselsäure zu synthetisieren. Dabei werden die Alkoxygruppen des Silans nach und nach durch OH-Gruppen substituiert. Bei dieser exothermen Reaktion entstehen Orthokieselsäure und Methanol. Aufgrund sich anschließender Reaktionen kann dieser Arbeitsschritt sowohl als Pre-Hydrolyse als auch als Pre-Polymerisation aufgefasst werden. Um Verwechslungen vorzubeugen, soll die erhaltene Lösung darum als pre-polymerisiertes TMOS (PP-TMOS) bezeichnet werden. Für die Herstellung verschieden konzentrierter PP-TMOS-Lösungen wurden jeweils entsprechend berechnete Volumina der reinen TMOS-Lösung, entionisiertes Wasser und als Katalysator Salzsäure vermischt, so dass sich letztendlich eine Konzentration von 0,01 M HCl einstellte. In der Praxis wurde zunächst das entionisierte Wasser zusammen mit einem Magnetrührer in ein Becherglas gegeben. Anschließend wurde die TMOSLösung zugegeben und der gesamte Ansatz im Kühlschrank auf 4°C gekühlt. Danach wurde der Ansatz auf einer Rührplatte durchmischt und innerhalb einer Minute das berechnete Volumen der Salzsäure tropfenweise zupipettiert, woraufhin das Becherglas mit Parafilm abgedeckt wurde. Nachdem die Flüssigkeit unter ständigem Rühren wieder klar wurde und eine Temperatur von etwa 50°C erreicht hatte, wurde der Ansatz unverzüglich in einen Gefrierschrank bei -20°C gestellt. Nach zehn Minuten war die Temperatur des Ansatzes auf ca. 4°C gekühlt und die Lösung wurde für die weitere Verwendung bei 4°C aufbewahrt.

Die Gelbildung selbst wurde in einem zweiten Schritt durch die Zugabe entsprechender Pufferlösungen beschleunigt. Das Gesamtvolumen der Ansätze für die Hydrogele wurde in der Regel auf 1,5 ml festgelegt, wobei nach Herstellung entsprechend konzentrierter Ausgangslösungen Endkonzentrationen von 25, 50 und 75 mg Kollagen, sowie 0,50, 0,75 und 1,00 M TMOS pro Ansatz eingestellt wurden. Das Vermischen der beiden Komponenten in einem Zentrifugenröhrchen musste intensiv, aber auch zügig erfolgen, da sich die Gelbildungsdauer bei hohen Kollagen- bzw. TMOS-Konzentrationen auf teilweise wenige Sekunden verkürzte. Solange die Mischungen noch fließfähig waren, wurden sie zur Vermeidung von Blasenbildung direkt in die Vertiefungen von 24er Wellplatten gegossen. Nach wenigen Minuten wurde auch bei den niedrig-konzentrierten Ansätzen der Gelpunkt erreicht.

Für die zur Festigung der Festkörperstruktur durchgeführte Alterung wurden die reinen Silikat- und Hybridgele direkt nach Vermischung der Komponenten und Erreichen des Gelpunktes luftdicht abgeschlossen und für 48 Stunden bei 37°C gelagert. Anschließend wurde entsprechend dem angestrebten Produkt weiterverfahren.

Die Versuche ergaben, dass sich brauchbare reine Silikatgele unter den gewählten Randbedingungen (Raumtemperatur, pH7,4) erst ab einer Konzentration von 1,0 M TMOS (in 0,1 M Tris/HCl-Puffer) bildeten. Die so erhaltenen Hydrogele dienten im Folgenden als kollagenfreie Referenz. Der Zusammensetzungsbereich der, durch Mischung von homogenen Kollagensuspensionen und hydrolysierten TMOS-Lösungen erhaltenen, Hybrid-Hydrogele, wurde durch die jeweilige Kombination der abgestuften Endkonzentrationen von TMOS (0,25, 0,50, 0,75, 1,00 M) und Chondrosia-Kollagen (25, 50, 75 mg/ml) abgedeckt. Dabei wurde jede der angegebenen TMOS-Konzentrationen mit jeder der Kollagenkonzentrationen kombiniert. Höher konzentrierte Kombinationen waren nicht möglich, da der Gelpunkt entsprechender Ansätze bereits nach wenigen Sekunden erreicht wurde und somit eine ausreichend intensive Vermischung beider Komponenten nicht gewährleistet werden konnte. Die auf diese Weise erhaltenen Silikat- und Hybrid-Hydrogele stellten selbst ein Zwischenprodukt sowie den Ausgangspunkt für die Weiterverarbeitung zu Hybridmaterialien mit anderen Eigenschaften dar.

**Ausführungsbeispiel 3**

**Silikat- und Kollagen-Silikat-Hybrid-Xerogele**

Ausgehend von den in Ausführungsbeispiel 2 beschriebenen Silikat- und Hybrid-Hydrogelen (V=1,5 ml), wurden diese unter verschiedenen klimatischen Bedingungen getrocknet. Variiert wurden dabei Temperatur (4°C, 21°C, 37°C, 60°C) und Luftfeuchtigkeit (ständiger Luftabzug, abgeschlossenes Volumen). Die besten Ergebnisse wurden durch langsame Trocknung bei Raumklima erhalten. Dazu wurden die gealterten Proben vorsichtig aus den Wellplatten gelöst, indem sie nach Einbringen einer kleinen Bohrung mit Druckluft von unten herausgedrückt wurden. Die somit unbeschädigten Proben wurden anschließend in Petrischalen ausgelegt und langsam (ca. 2-3 Tage) luftgetrocknet.

Die Herstellung von Xerogelen ging generell von den in Ausführungsbeispiel 2 beschriebenen Hydrogelen aus. Der dafür ausgeschöpfte Zusammensetzungsbereich von TMOS und Kollagen konnte dabei mit leichten Einschränkungen beibehalten werden. So wurde festgestellt, dass die Chondrosia-Kollagenkonzentrationen in Kombination mit entsprechenden TMOS-Konzentrationen einen entscheidenden Einfluss auf das Trocknungsverhalten der Hydrogele und damit auf die Eigenschaften der entsprechend erhaltenen Xerogele ausüben. Mit dem Ziel, möglichst formtreue Xerogele zu erhalten, wurden verschiedene Verfahren und Bedingungen getestet, um die flüssige Phase aus den Hydrogelen zu entfernen.

Die Ergebnisse der Trocknung durch Gefriertrocknung der Hydrogele zeigten bei konstanter TMOS-Konzentration einen deutlichen Einfluss der Alterungsdauer im Rahmen der Lufttrocknung und des eingelagerten Kollagenanteils. Wie in **Fig. 6** beispielhaft anhand einer TMOS-Konzentration von 1,00 M gezeigt ist, führt die Gefriertrocknung nach einstündiger Lufttrocknung durch die Volumendehnung der Flüssigphase beim Einfriervorgang, aufgrund des nur schwach ausgebildeten Silikatnetzwerks zur vollständigen Zerstörung desselben mit dem Ergebnis eines feinen Pulvers (Teilbild (a)). Außerdem führen Kapillarkräfte zu inneren Spannungen was ebenfalls zur Zerstörung und Schrumpfung des trocknenden Gels beiträgt. Mit zunehmender Dauer der Lufttrocknung und somit dem Fortschreiten der Alterung, wird das Silikatnetzwerk stabilisiert, wodurch nach Gefriertrocknung zunehmend größere Silikatfragmente des dennoch zerstörten Silikatgels erhalten wurden (Teilbilder (b) und (c)).

Im Gegensatz dazu zeugten bei den Hybridgelen nur wenige makroskopisch erkennbare Risse von der Schädigung des Silikatnetzwerkes durch die Gefriertrocknung, wie die Teilbilder (a), (b) und (c) der **Fig. 7** erkennen lassen. Hier sind die zum vorhergehenden Beispiel entsprechend äquivalenten Zustände bei Konzentrationen von 0,50 M TMOS und 50 mg/ml Chondrosia-Kollagen dargestellt. Es wurde festgestellt, dass die Schädigungen mit steigender Kollagenkonzentration immer geringer ausfielen. Man kann daraus schlussfolgern, dass die eingelagerten Kollagenfibrillen offenbar einen Teil der auf tretenden Spannungen aufnehmen, Risse umlenken bzw. das Risswachstum stoppen. Hier ist bereits der deutliche Einfluss des Kollagens auf die mechanischen Eigenschaften des Hybridmaterials erkennbar. Die auf diese Weise erhaltenen Xerogele zeigten jedoch stets deutliche Schrumpfung (vgl. Teilbilder (a) und (c)) und konzentrationsabhängige Deformation, was auf entsprechende Kapillarkräfte beim Trocknungsvorgang schließen lässt. Die langsame Trocknung der Proben an Luft bei Raumtemperatur brachte hingegen befriedigende Resultate. So führten TMOS-Konzentrationen ab etwa 0,75 M und steigend bei reinen Silikatgelen zu makroskopisch rissfreien Xerogelen (siehe Teilbild (d) in **Fig. 6**). Entscheidend wirkte sich auch hier die Einlagerung des Chondrosia-Kollagens aus, mit dessen steigendem Anteil auch bei niedrigen TMOS-Konzentrationen rissfreie Xerogele erhalten wurden (siehe Teilbild (d) in **Fig. 7**). Die Formtreue verbesserte sich jeweils mit steigender TMOS- und Kollagenkonzentration. Modifikationen wie die Trocknung im Wärmeschrank bei 37°C oder Trocknung unter dem Abzug konnten die Qualität der Xerogele bezüglich der genannten Eigenschaften nicht entscheidend erhöhen. Die Ergebnisse ließen sich unter Nutzung eines Klimaschranks, bei dem Temperaturregime und Luftfeuchte gezielt geregelt werden können, wahrscheinlich noch optimieren [Kortesuo, P.: Sol-gel derived silica gel monoliths and microparticles as carrier in controlled drug delivery in tissue administration. (2001). - Dissertation]. Dabei würden möglicherweise die Formtreue weiter verbessert und Konzentrationen (TMOS, Kollagen) weiter gesenkt werden können.

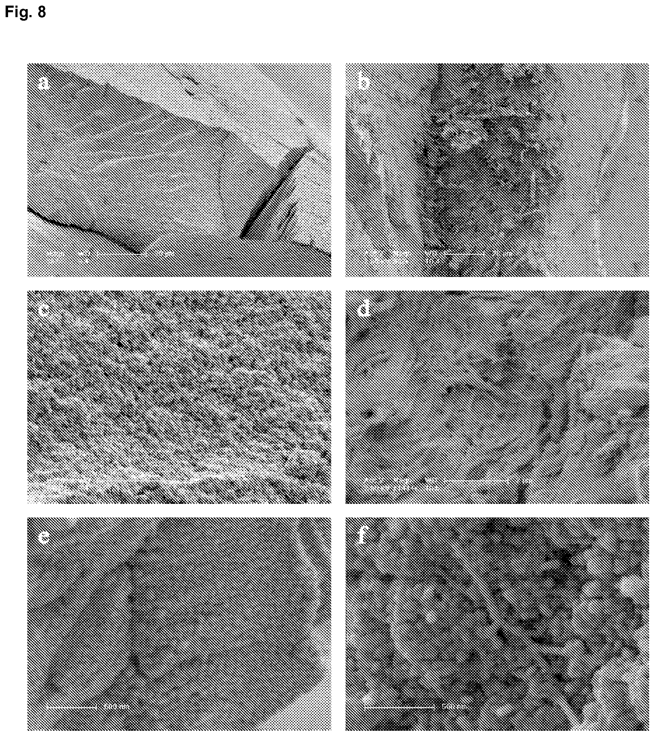

Dreidimensional ortsauflösende Messungen der Xerogele mittels µCT waren aufgrund der hohen Absorption des dichten Materials mit der zur Verfügung stehenden Energie (11 keV) nicht möglich. Deshalb wurde für die vergleichenden Untersuchungen des strukturellen Aufbaus der reinen Silikat-Xerogele und Hybrid-Xerogele (Silikat und Chondrosia-Kollagen) die Rasterelektronenmikroskopie genutzt. In der **Fig. 8** sind einige ausgewählte Aufnahmen gegenübergestellt. Die linken Teilbilder zeigen dabei ein Silikat-Xerogel, das durch Lufttrocknung einer 1,0 M PP-TMOS-Lösung in 0,1 M Tris/HCl-Puffer pH 7,4 erhalten wurde. Die Teilbilder des Hybrid-Xerogels auf der rechten Seite enthalten den gleichen TMOS-Anteil, wobei anstelle der Pufferlösung eine homogene Chondrosia-Kollagensuspension (50 mg/ml) eingesetzt wurde. Die bei kleiner Vergrößerung aufgenommenen Teilbilder (a) und (b) stellen typische Bruchflächen der beiden Geltypen gegenüber. Während beim reinen Silikat-Xerogel eine verhältnismäßig glatte unstrukturierte Oberfläche der Bruchkante zu erkennen ist, zeigt sich beim Hybrid-Xerogel ein anderes Bild. Hier offenbart sich bereits der zweikomponentige Materialverbund, indem aus der rauen Bruchfläche silikatisierte Kollagenfasern hervortreten. Im Teilbild (c) wird der Aufbau des Xerogels aus der engen Zusammenlagerung einzelner Silikatpartikel etwa gleicher Größe sichtbar. Im Gegensatz dazu ist beim Hybrid-Xerogel (d) eine annähernd gleichmäßige Verteilung von Kollagenfibrillen und -fasern in der Silikatphase erkennbar. Außerdem lässt sich wiederum feststellen, dass es sich dabei nicht nur um eine mehr oder minder lockere physikalische Einlagerung des Kollagens, sondern vielmehr um eine innige Verbindung beider Komponenten handelt. Bei höherer Vergrößerung wird, wie die Teilbilder (e) und (f) verdeutlichen, bei beiden Geltypen eine dichte Anordnung der einzelnen Silikatpartikel beobachtet. Die Bruchfläche des reinen Silikat-Xerogels erscheint dabei sehr sauber und eben, wohingegen die des Hybridmaterials stärker zerklüftet ist. Die einzelnen Kollagenfibrillen sind dabei vollständig in das Silikatnetzwerk integriert.

Die Charakterisierung der makroskopischen mechanischen Eigenschaften der Xerogele erfolgte nach einer Abwandlung des in DIN 1048 Teil 5 beschriebenen Versuchs zur Bestimmung der Spaltzugfestigkeit von Beton. Da man auch bei den Xerogelen aufgrund des hohen Silikatanteils von einem spröden Materialverhalten ausgehen kann, wurde diese Technik in der in Abschnitt 6.3 von Ausführungsbeispiel 6 beschriebenen Weise übernommen. Als Vorteil gegenüber einem konventionellen Druckversuch wurde angesehen, dass hier die gesamte Probengeometrie bei der Berechnung berücksichtigt wird und somit zu qualitativ vergleichbaren Resultaten führen sollte. Ziel dieser Testungen war es, den Einfluss des Kollagenanteils in Verbindung mit der eingesetzten TMOS-Konzentration zu charakterisieren.

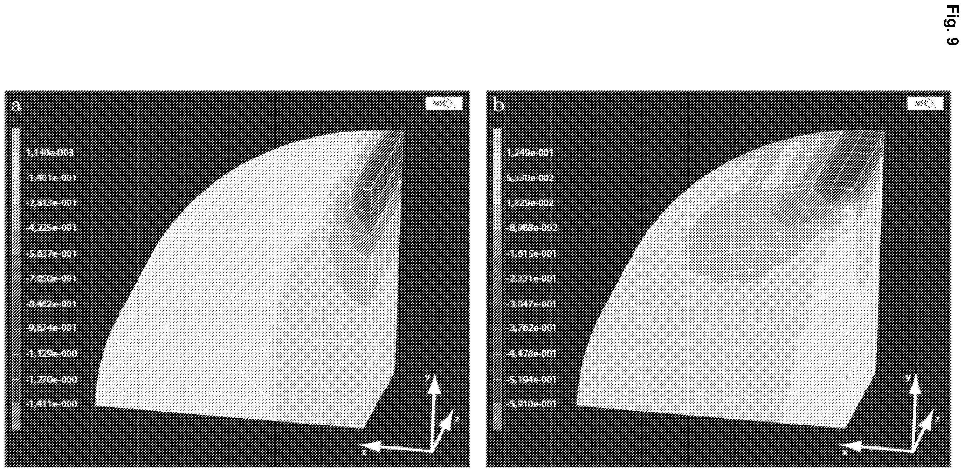

Zunächst wurde eine Finite-Elemente-Simulation des Versuchs zu Bestimmung der Spaltzugfestigkeit anhand eines, wie in Abschnitt 6.3 von Ausführungsbeispiel 6 beschrieben, angepassten Modells durchgeführt. Besonderes Interesse lag auf der Visualisierung der Spannungsverteilung im Probenvolumen die für das Versagen durch Bruch verantwortlich sein würde. Die für den vorliegenden Versuch bedeutsamen Ergebnisse der Simulation sind in **Fig. 9** dargestellt. Die Zahlenangaben sind hier aufgrund der hypothetischen Materialparameter und idealisierten Modellgeometrie ausschließlich als qualitatives Ergebnis zu verstehen. Das Teilbild (a) zeigt die Spannungsverteilung längs zur Druckachse (y-Richtung). Das Druckspannungsmaximum ist dabei in der Nähe der Belastungslinie lokalisiert und schwächt sich zum Inneren der Probe um etwa eine Größenordnung ab. Entscheidend für das Versagen der Probe unter steigender Druckbelastung ist jedoch die, aufgrund der Probengeometrie in x-Richtung resultierende Zugspannung, deren Verteilung im Teilbild (b) dargestellt ist. Diese erreicht ihr Maximum im Inneren der Probe, wobei sich eine H-förmige Spannungsebene längs der Belastungsachse ausbildet. In abgeschwächter Form steht das gelb dargestellte Probenvolumen ebenfalls unter Zugspannung. In Richtung der Belastungslinie geht die Zugspannung in eine Druckspannung über. Der Vergleich beider Teilresultate ergibt, dass die maximale Zugspannung in x-Richtung etwa eine Größenordnung unter der maximalen Druckspannung in y-Richtung liegt.

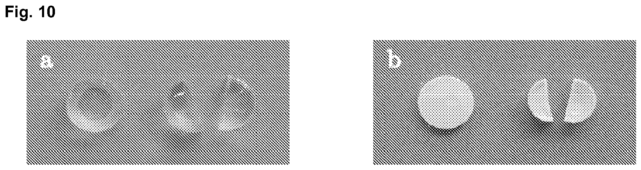

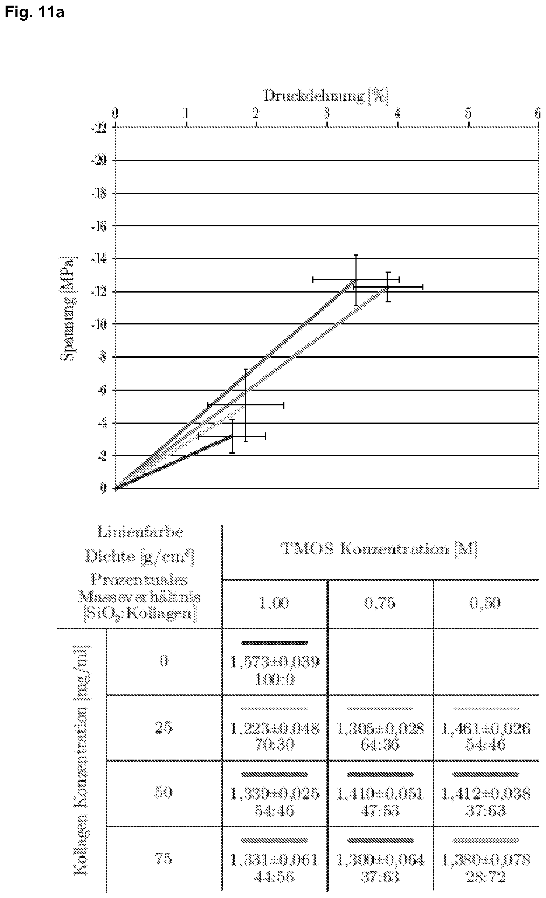

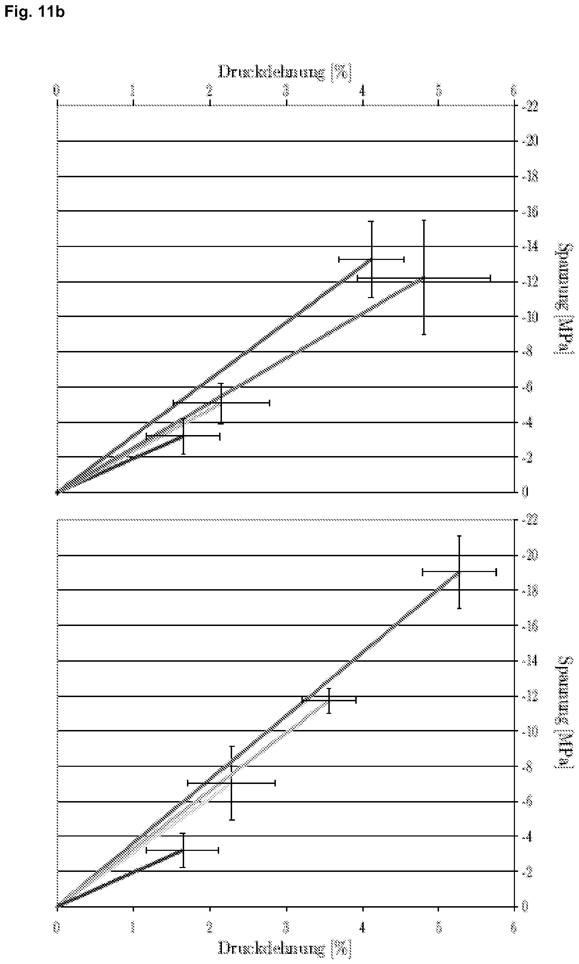

Bei den, nach der in Abschnitt 6.3 von Ausführungsbeispiel 6 beschriebenen Vorgehensweise durchgeführten, mechanischen Testungen der Xerogele, zeigte sich das nach den Ergebnissen der Finite-Elemente-Simulation erwartete Bruchverhalten. Bei Belastung der reinen Silikatbzw. Hybrid-Xerogele über die Elastizitätsgrenze hinaus, führt die hohe Zugspannung entlang der Mittelebene der Proben zum senkrechten Zerbrechen in zwei etwa gleichgroße Teilstücke (siehe **Fig. 10**). Um den Einfluss des Chondrosia-Kollagens auf die mechanischen Eigenschaften zu untersuchen, wurden die elastischen Eigenschaften entsprechender Hybrid-Xerogele mit denen reiner Silikat-Xerogele verglichen. Dazu wurden die Xerogele nach dem erläuterten Prüfregime bis zum Bruch belastet. Die Auswertung der dabei erhaltenen Messwerte erfolgte nach der in Abschnitt 6.3 von Ausführungsbeispiel 6 beschriebenen Vorgehensweise, so dass letztendlich für alle getesteten Zusammensetzungen entsprechend vergleichbare Daten erhalten wurden. Die Ergebnisse sind anhand der Bruchdehnung und Spaltzugfestigkeit in den drei Teildiagrammen der **Fig. 11** aufgetragen. In allen Spannungs-Dehnungs-Diagrammen ist zum Vergleich das elastische Verhalten der reinen Silikat-Xerogele (1,00 M TMOS) bis zur Elastizitätsgrenze eingezeichnet. Die zusammensetzungsabhängige Änderung dieser Eigenschaften zeigen die jeweils andersfarbig dargestellten Kurven. Diese repräsentieren die entsprechenden Hybrid-Xerogele, deren mittlere Dichten und Masseverhältnisse (SiO$_2$:Kollagen) in der Tabelle links unten zusammengefasst sind. Das Diagramm links oben in **Fig. 11** enthält die elastischen Daten der auf einer TMOS-Konzentration von 1,00 M basierenden Xerogele mit Kollagenkonzentrationen von 25, 50 bzw. 75 mg/ml. Bei gleicher Variation der Kollagenkonzentrationen sind im Diagramm rechts oben in **Fig. 11** die elastischen Eigenschaften der Hybrid-Xerogele mit 0,75 M TMOS, im Diagramm rechts unten in **Fig. 11** mit 0,50M TMOS dargestellt.

Wie den Diagrammen zu entnehmen ist, liegt die Spaltzugfestigkeit der reinen Silikat-Xerogele (1,00 M TMOS) bei etwa 3,5 MPa, wobei eine Bruchdehnung von etwa 1,5 % gemessen wurde. Beim Einsatz derselben TMOS-Konzentration wirkt sich das Vorhandensein von 30 Masse-% Chondrosia-Kollagen (25 mg/ml) bereits in einer leichten Verschiebung der Spaltzugfestigkeit und Bruchdehnung zu höheren Werten aus. Deutlich verbesserte Eigenschaften zeigen sich bei den Hybrid-Xerogelen mit 1,00 M TMOS und 50 mg/ml Chondrosia-Kollagen, was hier einem Masseverhältnis von 54:46 entspricht. Bei diesen Proben wurde die mittlere Spaltzugfestigkeit auf etwa 13 MPa bei einer Bruchdehnung von etwa 3,5 % bestimmt, was eine Steigerung gegenüber den reinen Silikatgelen um etwa den Faktor 4,3 bzw. 2,3 bedeutet. Die Konzentrationserhöhung des Kollagens auf 75 mg/ml scheint darüberhinaus keine weiteren Änderungen der elastischen Eigenschaften zu bewirken. Beim Vergleich der anhand der Anstiege der dargestellten Kurven abzulesenden Elastizitätsmoduln zeigt sich, dass unter den beschriebenen Bedingungen scheinbar keine Beeinflussung derselben durch den Kollagenanteil zu verzeichnen ist. Um eine weitere Verschiebung der Masseverhältnisse zugunsten des Kollagenanteils zu erreichen, aber dennoch eine gute Verarbeitbarkeit des Materials zu beizubehalten, wurde bei der Gel-Herstellung die TMOS-Konzentration herabgesetzt. Die Ergebnisse der mechanischen Testungen der entsprechend mit 0,75 M TMOS angesetzten Hybrid-Xerogele sind im rechts oben ersichtlichen Diagramm in **Fig. 11** aufgetragen. Die Kurven bestätigen die bereits festgestellten Eigenschaftsänderungen mit zunehmendem Kollagenanteil. Während bei 25 mg/ml Chondrosia-Kollagen im Vergleich zum reinen Silikat-Xerogel nur leichte Steigerungen der Spaltzugfestigkeit und Bruchdehnung festzustellen sind, werden bei 50 und 75 mg/ml Kollagen ganz eindeutige Veränderungen der elastischen Eigenschaften erzielt. So steigen die mittleren Spaltzugfestigkeiten auf etwa 12 bzw. 13MPa und die Bruchdehnungen auf 4 bzw. 5 %. Wiederum kann keine eindeutige Abhängigkeit der Elastizitätsmoduln von der Kollagenkonzentration erkannt werden. Als untere Grenze der eingesetzten TMOS-Konzentration wurde 0,50 M gewählt, da sich hier auch bei der niedrigsten Kollagenkonzentration (25 mg/ml) unter den festgelegten Randbedingungen gerade noch formtreue Hybrid-Xerogele für die mechanischen Testungen herstellen ließen. Im Vergleich zu den zuvor beschriebenen Verhältnissen, zeigt sich bereits bei 25 mg/ml Chondrosia-Kollagen, was einem Masseverhältnis von 54:46 entspricht, eine deutliche Erhöhung der Spaltzugfestigkeit auf etwa 7 MPa bei einer Bruchdehnung von leicht über 2 %. Die Steigerung des Kollagenanteils auf 63 Masse-% durch die Zugabe von 50 mg/ml Chondrosia-Kollagen resultiert hier in der in dieser Arbeit deutlichsten Eigenschaftsänderung des Hybridmaterials gegenüber dem reinen Silikat-Xerogel. Mit einer Spaltzugfestigkeit von etwa 19 MPa liegt diese um etwa den Faktor 6,3 über der kollagenfreien Referenzprobe. Parallel dazu steigert sich auch die Bruchdehung um etwa den Faktor 3,3 auf über 5 %. Unter den in dieser Arbeit gewählten Bedingungen fallen jedoch bei der nochmaligen Erhöhung des Kollagenanteils (75 mg/ml) und einem Masseverhältnis von 28:72 beide Kennwerte wieder deutlich zurück, liegen dabei aber dennoch weit über denen des reinen Silikat-Xerogels.

Anhand der vorliegenden Ergebnisse lässt sich festhalten, dass in allen geschilderten Fällen eine deutliche Verbesserung der elastischen Eigenschaften der Xerogele durch das Vorhandensein des eingelagerten Chondrosia-Kollagens erzielt werden konnte. Beim übergreifenden Vergleich aller Messergebnisse ist aber auch festzustellen, dass die charakterisierenden elastischen Eigenschaften scheinbar nicht ausschließlich vom Masseverhältnis der beiden Einzelkomponenten abhängen, sondern auch von deren jeweiliger Konzentration im angesetzten Gesamtvolumen. So wirkt sich diese nach den vorliegenden Erkenntnissen nicht zuletzt auf den Silikatisierungs- und Trocknungsprozess aus. Es hat sich gezeigt, dass ausgehend vom reinen Silikat-Xerogel niedrige Kollagenkonzentrationen (25 mg/ml) zunächst nur geringe Erhöhungen der Spaltzugfestigkeiten und Bruchdehnungen zur Folge haben. Bei Verdoppelung des Anteils steigen beide Kennwerte dann jedoch um ein jeweils Vielfaches. So werden hier bei einem Masseanteil von 63 % Chondrosia-Kollagen die höchsten gemessenen Spaltzugfestigkeiten und Bruchdehnungen erreicht. Die nochmalige Erhöhung der Kollagenkonzentration (75 mg/ml) ruft hingegen nur geringe zusätzliche Kennwertänderungen bei den Xerogelen mit 1,00 und 0,75 M TMOS hervor. Bei der dabei niedrigsten TMOS-Konzentration (0,50 M), also den Xerogelen mit dem mit 72 % höchsten Kollagenanteil, ist sogar wieder ein deutlicher Rückgang der Spaltzugfestigkeit und Bruchdehnung festzustellen. Dieser Abfall lässt sich möglicherweise mit dem Überschreiten eines als optimal einzustufenden Masseverhältnisses von Silikat zu Kollagen erklären. So konnte die Silikatisierung des Kollagens möglicherweise nicht ausreichend erfolgen und sich somit das Potenzial des Verbundmaterials nicht vollständig entfalten. Interessanterweise stimmt das in diesen Versuchen als optimal erkannte Masseverhältnis von 37% Silikat zu 63% Chondrosia-Kollagen nahezu genau mit dem bei der Untersuchung der Spikulen des Glasschwammes Hyalonema sieboldi spektroskopisch ermittelten Kollagenanteil (64 Masse-%) überein [Ehrlich, H.; Hanke, T.; Simon, P.; Goebel, C.; Heinemann, S.; Born, R. ; Worch, H.: Demineralization of natural silica based biomaterials: new strategy for the isolation of organic frameworks. In: BIOmaterialien 6 (2005), Nr. 4, S. 297-302].

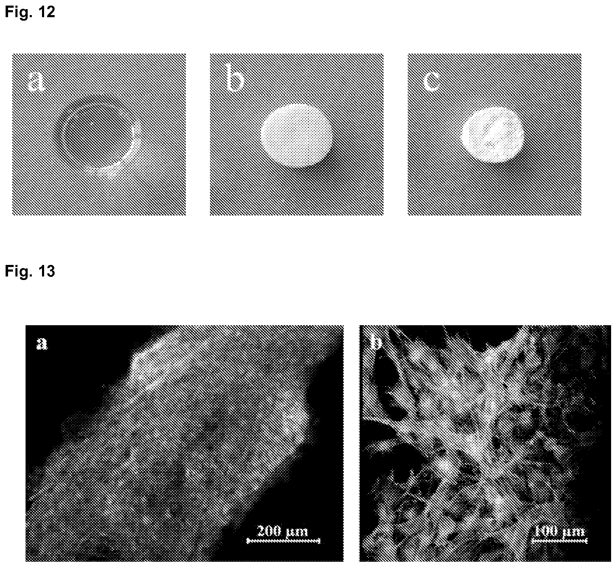

Für die zellbiologischen Untersuchungen wurden neben den bisher betrachteten reinen Silikat-Xerogelen und Hybrid-Xerogelen mit Chondrosia-Kollagen, äquivalente Proben mit Kalbshautkollagen Typ I hergestellt. Die dazu als Ausgangsmaterial verwendete Tropokollagenlösung wurde zunächst einigen aufreinigenden Schritten unterzogen. Die Reassemblierung des Tropokollagens zu Fibrillen wurde im Hinblick auf die resultierende Fibrillenausbeute und -morphologie in entsprechenden Voruntersuchungen in Abhängigkeit zweier Pufferlösungen (Puffer A und B) mit jeweils unterschiedlichen Ionenstärken charakterisiert. Die Kinetik der Fibrillenbildung wurde turbidometrisch verfolgt, wobei keine wesentlichen Unterschiede zwischen den beiden Pufferlösungen festgestellt werden konnten. Mittels einer Proteinbestimmung nach Lowry wurde der Anteil des zu Fibrillen umgesetzten Tropokollagens bei Puffer A auf 77,3 %, bei Puffer B auf 65,7% bestimmt. Atomkraftmikroskopische Untersuchungen ergaben, dass bei beiden Pufferlösungen gleichfalls sehr gleichmäßige Fibrillen gebildet wurden. Da sich die Morphologie der Fibrillen nicht unterschied, wurde mit dem Ziel, eine möglichst hohe Ausbeute an Fibrillen für die Herstellung der materialaufwändigen Xerogele zur Verfügung zu haben, im weiteren Verlauf die Pufferlösung A verwendet. Jeweils exemplarische Proben der für die folgenden Untersuchungen eingesetzten reinen Silikat-Xerogele (a) sowie der Hybrid-Xerogele auf sehen. Alle drei genannten Xerogel- Basis von Chondrosia-Kollagen (b) bzw. Kalbshaut-Kollagen Typ I (c) sind in **Fig. 12** zu Typen sowie die zum Vergleich mitgeführten Beschichtungen mit Chondrosia- bzw. Kalbshautkollagen Typ I (eingetrocknete Kollagenlösungen in Polystyrol-Wellplatten) wurden wie in Abschnitt 7.4 von Ausführungsbeispiel 7 beschrieben mit Mausosteoblasten besiedelt. Die Beurteilung der Biokompatibilität wurde im Folgenden zunächst anhand von Proliferationsuntersuchungen mittels MTT-Test vorgenommen. Es stellte sich jedoch heraus, dass verlässliche quantitative Messungen diesbezüglich auf den reinen Silikat-Xerogelen nicht realisierbar waren. Aufgrund der mangelnden mechanischen Stabilität dieser Xerogele, die bereits im trockenen Zustand häufig zur Rissbildung unter Eigenspannungen führte, verstärkte sich dieser negative Effekt bei der Lagerung in Zellkulturmedium. Möglicherweise führten auch hier Kapillarkräfte zu lokalen Spannungen die das Xerogel in mehrere Bruchstücke zerfallen ließen. Aus diesem Grund ließen sich keine einheitlichen Probenoberflächen gewährleisten, was zu Beginn der Besiedlung zu einer uneinheitlichen Anzahl adhärenter Zellen und somit auch zu verfälschten Ergebnissen bei der Bestimmung der Zellzahl nach bestimmten Zeitpunkten der Kultivierung geführt hätte. Somit dienten ausschließlich qualitative Untersuchungen mittels Fluoreszenz- und Laser- Scanning-Mikroskopie zur Beurteilung des Zellverhaltens auf den reinen Silikat-Xerogelen. Zwei entsprechende Aufnahmen nach 7-tägiger Kultivierungsdauer sind in **Fig. 13** zu sehen. In der im Teilbild (a) dargestellten Fluoreszenz-Aufnahme lässt sich erkennen, dass sich auf dem Xerogel innerhalb der Kulturdauer ein dichten Zellrasen gebildet hat. Die deutliche Abgrenzung desselben ist vermutlich auf die glatte aber unebene Probenoberfläche zurückzuführen, was zum Zusammenrutschen der Zellen bei der Besiedlung geführt hat. Bei höherer Vergrößerung (LSM-Aufnahme in Teilbild (b)) wird die vollständige Adhärenz und bereits teilweise Überlagerung der Zellen deutlich.

Die verbesserten mechanischen Eigenschaften der Hybrid-Xerogele auf Basis des Chondrosia-Kollagens bestätigten sich auch bei den mehrtägigen Zellkulturtests. Im Gegensatz zu den reinen Silikat-Xerogelen behielten sie während des gesamten Versuchszeitraums vollständig ihre Form und Handhabbarkeit, wodurch auch quantitative Messungen möglich waren. Nach den Ergebnissen der Cytotoxizitätstest mit dem Chondrosia-Kollagen (nicht mit Wasserstoffperoxid behandelt) musste davon ausgegangen werden, dass auch bei Besiedlung der entsprechenden Hybrid-Xerogele und Beschichtungen auf Polystyrol die entsprechend biologisch aktiven Substanzen ihre cytotoxische Wirkung ausüben. Diese Annahme bestätigte sich, indem bereits nach 24 Stunden mit keiner der beschriebenen Methoden Zellen auf den Hybrid-Xerogelen bzw. Beschichtungen festgestellt werden konnten. Entsprechend andere Ergebnisse sind bei Verwendung des mit Wasserstoffperoxid behandelten Chondrosia-Kollagens zu erwarten.

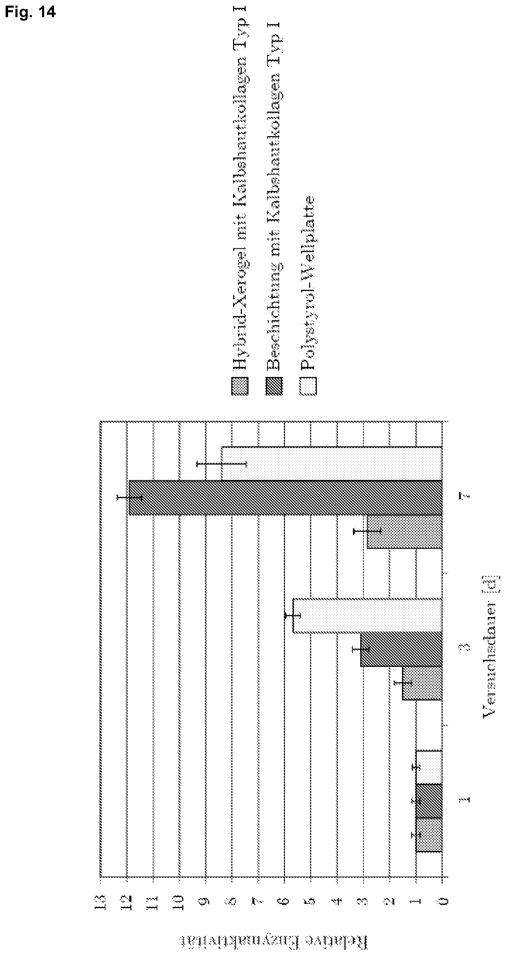

Als Alternative zum Chondrosia-Kollagen diente darum an dieser Stelle Kalbshaut-Kollagen Typ I als organische Komponente bei den zu testenden Hybrid-Xerogelen. Deren Herstellung erfolgte analog zu der bei den Xerogelen mit Chondrosia-Kollagen angewandten Vorgehensweise. Als geeignete Zusammensetzung hat sich dabei eine Kollagenkonzentration von 15 mg/ml bei 0,50 M TMOS herausgestellt, bei der Xerogele wie im Teilbild (c) der **Fig. 12** erhalten wurden. Diese waren wie die Xerogele auf Basis des Chondrosia-Kollagens bei den Zellkulturtests ebenso gut haltbar und handhabbar. Zunächst wurden diese Xerogele, als Vergleich entsprechende Kollagenbeschichtungen auf Polystyrol sowie Polystyrol-Wellplatten selbst mit Mausosteoblasten besiedelt und über einen Zeitraum von sieben Tagen kultiviert. Unter Verwendung des MTT-Tests wurden nach einem, drei und sieben Tagen die jeweiligen Enzymaktivitäten zur Quantifizierung der Zellproliferation gemessen. Diese sind bezogen auf die Aktivitäten am Tag 1 in **Fig. 14** dargestellt. Unter der Voraussetzung konstanter Enzymaktivität, können die angegebenen relativen Aktivitäten als Proliferationsfaktoren der am Tag 1 gesetzten Zellzahlen interpretiert werden. Wie im Diagramm zu erkennen ist, nimmt die Zellzahl auf den Hybrid-Xerogelen mit Kalbshautkollagen Typ I nach drei Tagen um etwa den Faktor 1,5 zu und verdreifacht sich etwa nach sieben Tagen. Bei Kultivierung auf den als ideales Referenzsystem zu sehenden Polystyrol-Wellplatten steigt die Zellzahl zu den entsprechenden Zeitpunkten um etwa den Faktor 5,5 bzw. 8,5. Auf den Beschichtungen mit dem auch für die Hybrid- Xerogele genutzten Kalbshautkollagen Typ I nimmt die Zellpopulation nach drei Tagen um etwa das Dreifache zu. Bemerkenswert ist im weiteren Verlauf die gegenüber den reinen Polystyrol-Wellplatten nochmals erhöhte Proliferation mit einem Faktor von etwa 12 nach sieben Tagen.

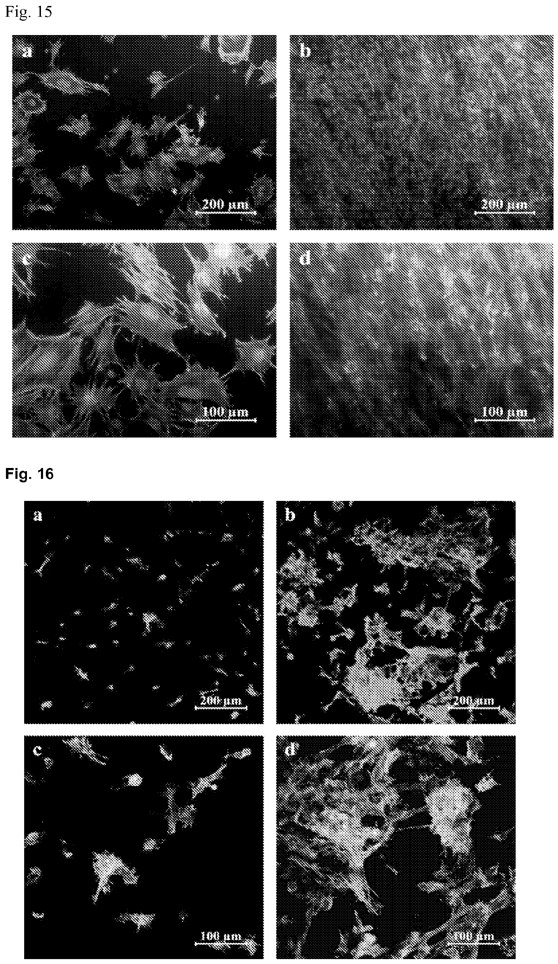

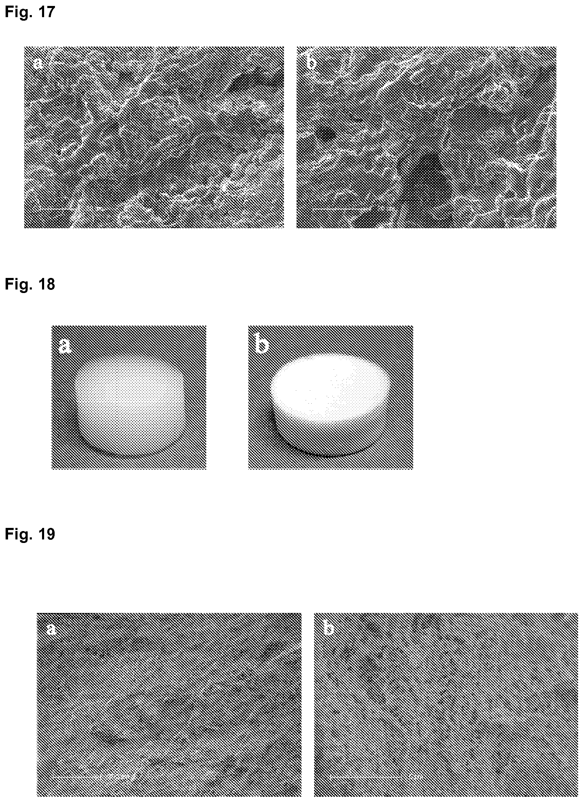

Neben den quantitativen Untersuchungen wurden entsprechende Proben der mit Mausosteoblasten besiedelten kollagenbeschichteten Wellplatten und Hybrid-Xerogele für die qualitative Beurteilung der Zelladhäsion und -proliferation mittels mikroskopischer Methoden präpariert. Diese sind in den Abschnitten 7.5 in Ausführungsbeispiel 7 (FM und LSM) und 6.2 in Ausführungsbeispiel 6 (REM) beschrieben. Aufgrund der ebenen Probenoberfläche konnten die fixierten und gefärbten Zellen auf den kollagenbeschichteten Wellplatten sehr gut mithilfe der Fluoreszenzmikroskopie untersucht werden. In **Fig. 15** sind entsprechende Aufnahmen nach 24 Stunden (Teilbilder (a) und (c)) und sieben Tagen (Teilbilder (b) und (d)) Kultivierung gegeben. Wie anhand der Aktingerüste zu sehen ist, sind die Zellen 24 Stunden nach der Besiedlung vollständig adhärent und haben bereits teilweise Kontakt untereinander hergestellt (Teilbilder (a) und (c)). In Übereinstimmung mit den zuvor erläuterten quantitativen Messungen ist die Zelldichte aufgrund intensiver Proliferation nach sieben Tagen Kulturdauer deutlich angestiegen, was anhand Zellkerne gut im Teilbild (b) zu sehen ist. Die vergrößerte Darstellung derselben Probe im Teilbild (d) zeigt außerdem, wie die Zellen zu diesem Zeitpunkt bereits eine geschlossene Zellschicht gebildet haben und teilweise sogar übereinander wachsen. Die dazu entsprechend äquivalenten Aufnahmen der auf den Hybrid-Xerogelen (Kalbshautkollagen Typ I) kultivierten Zellen sind in **Fig. 16** zusammengestellt. Aufgrund der rauen und unebenen Probenoberflächen der Xerogele konnten mittels Fluoreszenzmikroskopie keine befriedigenden Aufnahmen erstellt werden, weshalb hier zugunsten der Bildschärfe konfokale Laser-Scanning-Mikroskopie angewandt wurde. Im Teilbild (a) zeigt sich zunächst eine recht gleichmäßige Verteilung einzelner Zellen auf der Xerogel-Oberfläche. Der Farbkanal der Zellkerne ist hier aufgrund der starken Autofluoreszenz der Probe im entsprechenden Wellenlängenbereich und der in Verbindung mit der geringen Vergrößerung technisch bedingten schwachen Kerndarstellung ausgeblendet. Anhand der Aktingerüste lässt sich jedoch eine im Vergleich zu den beschichteten Wellplatten deutlich geringere Zelldichte feststellen. Wie auf den beschichteten Wellplatten sind die Zellen nach 24 Stunden ebenfalls adhärent, was anhand der ausgebreiteten Aktingrüste zu erkennen ist (Teilbild (c)). Die erkennbaren Morphologien der einzelnen Zellen und Zellhaufen sind jedoch sehr unterschiedlich und vielgestaltig, was auf die Anpassung an den jeweiligen Untergrund hinweist. Wie in den Teilbildern (b) und (d) zu sehen ist, steigt auch auf den Hybrid-Xerogelen innerhalb von sieben Tagen die Zelldichte durch Proliferation deutlich an, wobei sich geschlossene Zellinseln ausbilden, aber auch Oberflächenbereiche unbesiedelt bleiben. Diese Ergebnisse bestätigen somit die bereits anhand der quantitativen Messungen mittels MTT-Test belegte Zellproliferation. Um einen besseren Eindruck von der Oberfläche der Hybrid-Xerogele und der vermutlich davon bestimmten Zellmorphologie zu erhalten, wurde die bereits für das Teilbild (d) der **Fig. 16** genutzte Probe schonend an Luft getrocknet, mit Kohlenstoff beschichtet und mittels Rasterelektronenmikroskopie untersucht. Zwei der dabei angefertigten Aufnahmen der sieben Tage mit Mausosteoblasten besiedelten Probe sind in **Fig. 17** dargestellt. Wie in den Aufnahmen zu erkennen ist, wird das Hybrid-Xerogel selbst scheinbar aufgebaut aus einzelnen, mikrometergroßen Kompartimenten, die sich zu einem losen aber Oberfläche und überspannen Zwischenräume, so dass die Oberflächenstruktur des reinen Xerogels nur noch an wenigen Zwischenräumen sichtbar ist. Es lässt sich zusammenfassen, dass bei dem aus Chondrosia reniformis isolierten Kollagen wie erwartet auch nach einigen Aufbereitungsschritten eine cytotoxische Wirkung gegenüber Mausosteoblasten festgestellt wurde. Diese Cytotoxizität kann jedoch durch spezielle Behandlungsschritte (Behandlung mit Wasserstoffperoxid) deutlich reduziert werden. Die beschriebenen und nachgewiesenen biologischen Eigenschaften des Chondrosia-Kollagens und seiner Wirkstoffe können möglicherweise auf vielfältige Weise für biomedizinische Zwecke genutzt werden. So können z. B. die antibakteriellen Eigenschaften beim Einsatz des Kollagens in Zusammenhang mit Implantaten hilfreich sein. Die Besiedlung der reinen Silikat-Xerogele mit Mausosteoblasten 7F2 konnte mittels Fluoreszenz- und Laser-Scanning-Mikroskopie als erfolgreich bewertet werden.

Die mit Kalbshautkollagen Typ I hergestellten Hybrid-Xerogele ließen sich sehr gut für zellbiologische Untersuchungen verwenden. Hier konnte mittels quantitativer und qualitativer Methoden die Proliferation und gute Anpassung der Mausosteoblasten an die Probenoberfläche nachgewiesen werden.

**Ausführungsbeispiel 4**

**Herstellung von Silikat- und Kollagen-Silikat-Hybrid-Aerogelen**

Auch für die Silikat- und Hybrid-Aerogele bildeten die zugehörigen Hydrogele gemäß Ausführungsbeispiel 2 den Ausgangspunkt. Die anzuwendende Trocknung unter überkritischen Bedingungen erforderte zunächst eine Substitution der Flüssigphase (Wasser und Methanol) der Hydrogele. Dazu wurden die Hydrogele über fünf Tage in Reinstethanol eingelegt. Das Ethanol wurde dabei regelmäßig in Abständen von ca. 12 Stunden gewechselt. War dies erfolgt, wurde die Kritisch-Punkt-Trocknung in einem Autoklaven durchgeführt. Dabei wird das Ethanol zunächst bei einer Temperatur von etwa 7 °C unter Hochdruckbedingungen durch mehrmaliges Spülen durch flüssiges Kohlendioxid ersetzt. Anschließend wird die Temperatur auf 38 - 40°C erhöht, wobei das Kohlendioxid in den überkritischen Zustand übergeht, d. h. es existiert keine Grenzfläche mehr zwischen der flüssigen und gasförmigen Phase. Durch ein Ventil wird das überkritische Kohlendioxid bei Aufrechterhaltung der Bedingungen langsam abgeblasen. Durch das Vermeiden der Grenzfläche können keine Kapillarkräfte entstehen und die Festkörperstruktur der Probe bleibt unverändert erhalten. Nach Abschluss dieses Verfahrens wurden getrocknete Aerogele erhalten, deren innere und äußere Strukturen denen der Hydrogele entsprachen.

Die Herstellung von Aerogelen war aus allen hergestellten Hydrogelen möglich. Zwei Beispiele sind in **Fig. 18** dargestellt. Hier zeigt das Teilbild (a) ein reines Silikat- und Teilbild (b) ein Hybrid-Aerogel. Durch das schonende Entfernen der flüssigen Phase über eine Kritisch-Punkt-Trocknung wurde die innere Struktur sowie die äußere Gestalt der Hydrogele erhalten. Aufgrund der Probengröße mussten lange Diffusionswege einkalkuliert und darum die Substitutionsspülungen mit Ethanol bzw. flüssigem Kohlendioxid besonders intensiv durchgeführt werden. Vorhandene Restflüssigkeit im Inneren der Proben führte beim Trocknen sonst zu messbarer Schrumpfung.

Die Anfertigung der REM-Aufnahmen der Aerogele gestaltete sich aufgrund deren geringen elektrischen Leitfähigkeit sehr schwierig. Trotz entsprechender Bedampfung und zusätzlicher Stromableitungen mit Leitsilber führten Aufladungen bei der Mikroskopie zu schlechter Bildqualität. Dennoch konnten einige aussagekräftige Aufnahmen der Hybrid- Aerogele (0,5 M TMOS, 50 mg/ml Chondrosia-Kollagen) angefertigt werden, von denen zwei repräsentative Beispiele in **Fig. 19** zu sehen sind. Wie Teilbild (a) zum Ausdruck bringt, ist die Oberfläche des Hybrid-Aerogels unregelmäßig gestaltet aber dennoch recht kompakt. Bei höherer Vergrößerung wird die Porosität als Freiraum zwischen den silikatisierten Kollagenfibrillen und Silikatnetzwerken sichtbar (Teilbild (b)). Es kann aufgrund der speziellen Probenpräparation davon ausgegangen werden, dass die abgebildeten Strukturen auch dem Zustand des entsprechend vorausgegangenen Hydrogels entsprechen.

### Ausführungsbeispiel 5

### Herstellung von sphärischen Silikat- und Kollagen-Silikat-Hybrid-Partikeln

Zur Herstellung sphärischer Silikatpartikel und Hybridmaterialien wurden 0,1 MTris/HCl-Puffer (pH 7,4) bzw. entsprechend konzentrierte homogene Kollagensuspensionen (25, 50 und 75 mg/ml) auf Basis des Tris-Puffers (pH 7,4) tropfenweise in eine reine TMOS-Lösung pipettiert. Dabei bildete sich sofort eine Kugel aus, die nach 24 Stunden aus der Lösung genommen, gespült und luftgetrocknet wurde. Das resultierende Produkt entsprach somit einem sphärisch geformten Hybrid-Xerogel.

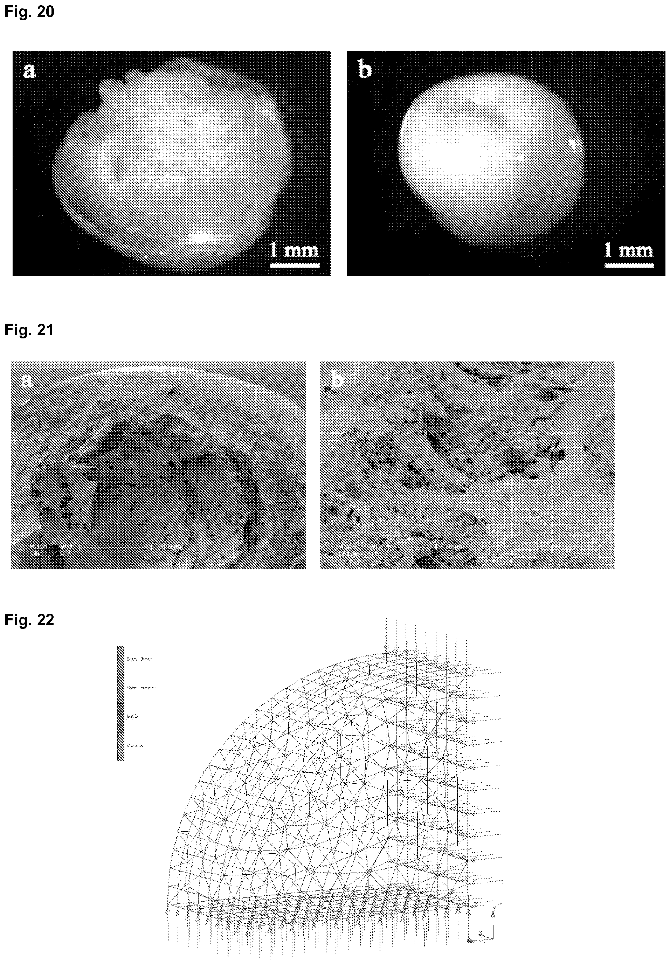

Beim Eintropfen von Lösungen neutralen pH-Werts in die 98 %ige TMOS-Lösung führte dabei vermutlich lokale Hydrolyse durch das Wasserangebot und spontane Polymerisation durch den erhöhten pH-Wert zur Ausbildung kugelförmiger Silikatpartikel. Auf diese Weise konnten unter Verwendung reinen Tris-Puffers reine Silikatpartikel und unter Verwendung homogener Chondrosia-Kollagensuspensionen Hybridpartikel in Lösung erhalten werden, von denen zwei repräsentative Beispiele in **Fig. 20** dargestellt sind. Die Trocknung der reinen Silikatpartikel führte jedoch, vermutlich infolge entsprechender Spannungen durch Kapillarkräfte, stets zum teilweisen Zerfall derselben (Teilbild (a)). Die mit entsprechend hoch konzentrierten Kollagensuspensionen hergestellten Partikel trockneten hingegen ohne sichtbare Schädigungen (siehe Teilbild (b)).

In **Fig. 21** sind REM-Aufnahmen eines Hybrid-Partikels dargestellt, wie es bereits im Teilbild (b) der **Fig. 20** zu sehen war. Anhand der in Teilbild (a) gezeigten REM-Aufnahme lässt sich der Verlauf der Silikatisierung gut nachvollziehen. Sobald der Tropfen der homogenen Chondrosia-Kollagensuspension (in diesem Beispiel 50 mg/ml) in die TMOS-Lösung eintaucht, bildet sich im Bestreben einer minimierten Oberflächenenergie eine Kugelform aus. Beginnend an der äußeren Kugeloberfläche wird das TMOS spontan in Kieselsäure hydrolysiert, die wiederum sofort am Kollagen polymerisiert. Da das TMOS von außen in die Kugel diffundieren muss, nimmt wegen des sich ausbildenden Konzentrationsgradienten der Grad der Silikatisierung ebenfalls von außen nach innen ab. Außerdem wird die Eindiffusion des TMOS durch Fortschreiten dieses Prozesses zunehmend erschwert. Da die Silikatpartikel als Bindeglieder zwischen einzelnen Kollagenfibrillen fungieren, wird das freie Kollagen zunehmends in kompakter Form von innen an die Mantelfläche angelagert, so dass am Ende im Inneren der Kugel ein Hohlraum verbleibt. Das Äußere des Partikels entspricht nach der Trocknung demzufolge einem Xerogel (siehe **Fig. 8**). Die hochaufgelöste Struktur im Inneren, wie aus Teilbild (b) entnommen werden kann, weist hingegen den Charakter eines silikatisierten Kollagenscaffolds auf (siehe Fig. 4).

### Ausführungsbeispiel 6

### Charakterisierung des mikro- und nanostrukturellen Aufbaus und der makroskopischen Eigenschaften

Mittels röntgenografischer und elektronenmikroskopischer Methoden wurde der mikro- und nanostrukturelle Aufbau, mittels mechanischer Testungen die daraus resultierenden makroskopischen Eigenschaften beurteilt.

### 6.1 Mikro-Computertomographie ($\mu$CT)

Die räumliche Verteilung des Silikats in den Hybrid-Scaffolds auf Basis von Chondrosia-Kollagen wurde mittels Mikro-Computertomographie ($\mu$CT) analysiert. Diese zerstörungsfreie Untersuchungsmethode kann, abhängig vom der Elementzusammensetzung und Kompaktheit der Probe, eine dreidimensionale Ortsauflösung vom Millimeter- bis zum Nanometer- Bereich ermöglichen. Die Messungen hierzu wurden unter Einsatz einer Energie von 11 keV durchgeführt.

### 6.2 Rasterelektronenmikroskopie (REM)

Die Rasterelektronenmikroskopie diente zur Charakterisierung des strukturellen Aufbaus der hergestellten Kollagenscaffolds, Silikat- und Hybridgele. Beim Auftreffen der Primärelektronen auf die Probenoberfläche bildet sich in Abhängigkeit von der Beschleunigungsspannung und dem Material der Probe ein Wechselwirkungsvolumen aus, aus dem die Sekundärsignale (u. a. Sekundärelektronen) stammen, die zur Figur detektiert werden können.

Von den zu untersuchenden Proben wurden mit einem Skalpell charakteristische Bereiche herausgetrennt und diese flach auf einem mit Kohlenstoffpad versehenen Probentisch fixiert. Alle Proben wurden daraufhin mit Gold bzw. Kohlenstoff bedampft und bei Bedarf eine Stromableitung mit Leitsilber gezogen um Aufladungen zu verringern. Alle REM-Aufnahmen wurden durch Detektion der Sekundärelektronen angefertigt. Es wurde dabei jederzeit mit Beschleunigungsspannungen von 1 - 5 kV gearbeitet um das Wechselwirkungsvolumen klein zu halten und Aufladungen zu minimieren.

### 6.3 Mechanische Testungen

Diese Testungen dienten der Beurteilung der zusammensetzungsabhängigen makroskopischen mechanischen Eigenschaften der hergestellten porösen Scaffolds und Xerogele, wobei unterschiedliche, der jeweiligen Aufgabenstellung angepasste Messmethoden verwendet wurden.

**Bestimmung der Elastizitätsmodulen der porösen Kollagenscaffolds**

Die mechanischen Testungen der reinen (40 mg/ml Chondrosia-Kollagen) und silikatisierten Scaffolds (zusätzlich 25, 50, 75 bzw. 100 mM TMOS), deren Herstellung in Ausführungsbeispiel 1 beschrieben ist, wurden an einer Universalprüfmaschine nach folgendem Versuchsregime durchgeführt:

    1. Relative Rampe auf 0,2 N (Axialdehnungsabgleich)
    2. Absolute Rampe auf 10% Druckdehnung mit 0,1 mm/s
    3. Entlastung

Pro Zusammensetzung wurden mindestens 10 der zylindrischen Proben (Durchmesser ca. 15 mm, Höhe ca. 12mm) aufrecht stehend unter Druckbelastung getestet. Anhand der gemessenen Daten wurden für die vergleichenden Betrachtungen die Elastizitätsmodul der Scaffolds ermittelt. Dazu wurde die gemessene Druckspannung über der Druckdehnung aufgetragen und mithilfe der Methode der kleinsten Quadrate der Elastizitätsmodul im Bereich bis 5 % Druckdehnung berechnet. In diesem Bereich verhielten sich alle getesteten Scaffolds elastisch. Um den Einfluss der TMOS-Konzentration darzustellen, wurden die ermittelten Elastizitätsmoduln gegeneinander in einem Diagramm aufgetragen.

**Finite-Elemente-Simulation des Druckversuchs zur Bestimmung der Spaltzugfestigkeit**

Um die beim Versuch zur Ermittlung der Spaltzugfestigkeit der Xerogele auftretenden Spannungsverteilungen in den Prüflingen zu beurteilen, wurde eine Simulation desselben mithilfe der Finite-Elemente-Methode (FEM) durchgeführt. Dafür wurde die Software Marc Mentat der Firma MSC verwendet. Zur Reduzierung des Rechenaufwandes empfahl es sich, Symmetrien des zu simulierenden Systems auszunutzen. Im vorliegenden Fall genügte es darum ein Viertel des gesamten Prüflings zu betrachten. Nach Festlegung der Grundgeometrie und Knotenpunkte wurde unter Verwendung von Dreieck-Elementen eine automatisierte Elementunterteilung vorgenommen und diese in z-Richtung erweitert. Das in **Fig. 22** dargestellte Gittermodell bestand somit aus 1071 Knoten und 1568 Elementen. Die Symmetriebedingungen wurden durch Unterbindung der Verschiebung der entsprechenden Schnittflächen-Knoten in x-Richtung (Sym. hor., grün) und y-Richtung (Sym. vert., türkis) berücksichtigt. Die Ganzkörperbewegung wurde durch Fixierung des Mittelpunkt-Knotens der Schnittlinie in alle drei Raumrichtungen unterbunden (GKB, blau). Als Simulation des Druckversuches wurde eine minimale Verschiebung der obersten Mantelknoten in negative y-Richtung als Lastfall vorgegeben (Druck, magenta).

**Bestimmung der Spaltzugfestigkeit bei den Xerogelen**

Nach der im vorherigen Abschnitt beschriebenen Finite-Elemente-Simulation zur Bestimmung charakteristischer Kennwerte für die mechanischen Eigenschaften der Xerogele, wurde das in DIN 1048 Teil 5 festgelegte Verfahren zur Ermittlung der so genannten Spaltzugfestigkeit auf das vorliegende System übertragen. Die Methode dient ursprünglich zur Beurteilung der Zugfestigkeit von Beton, da die Zugfestigkeit hier selbst nur schwierig zu messen ist und die Biegezugfestigkeit nicht vollständig befriedigt. Der Zusammenhang zwischen den drei genannten Festigkeiten ist noch nicht so weit geklärt, dass diese ineinander umgerechnet werden können. Die Spaltzugfestigkeit wird in der Regel an Betonzylindern von 100, 150, 200 oder 300 mm Durchmesser und einer Höhe von jeweils dem doppelten Durchmesser bestimmt. Zur Prüfung wird der Probekörper in eine Druckprüfmaschine gelegt und längs zweier genau gegenüberliegender gerader Linien belastet. Aus den gemessenen Kräften lässt sich die Spaltzugfestigkeit $\beta_{SZ}$( bei zylindrischer Probengeometrie mithilfe der Formel

$$(1) \qquad \beta_{SZ} = 2F/\pi dl$$

berechnen (F..gemessene Kraft, d..Durchmesser der Probe, l..Dicke der Probe).

Die üblichen Probenformen konnten aufgrund des sonst übermäßigen Materialaufwandes zwar nicht eingehalten werden, dennoch sollten bei einheitlicher Probengeometrie Vergleiche möglich sein, die den Einfluss des Komponentenverhältnisses Silikat:Kollagen charakterisieren. Die, wie in Ausführungsbeispiel 3 erläutert, hergestellten Xerogele dienten als Prüflinge, deren zylindrische Geometrie sich jeweils auf ca. 6 mm Durchmesser und 2 mm Dicke belief. Pro Zustand wurden mindestens 10 Proben gemessen. Eine graphische Darstellung des entsprechenden Versuchsaufbaus ist in **Fig. 23** gegeben. Die blau dargestellte Probe steht mit der Mantelfläche aufrecht auf dem feststehenden Unterstempel (grau), während durch die Translation des Oberstempels die Druckkraft aufgebracht wird. Das Prüfregime der Messungen lautete dabei wie folgt:

1. Relative Rampe auf 0,2 N (Axialdehnungsabgleich)
2. Absolute Rampe auf 20% Druckdehnung mit 0,1 mm/s
3. Entlastung

Als Ausgangswerte für die Auswertung dienten die gemessenen Druckkräfte und Druckdehnungen. Unter Berücksichtigung der jeweiligen Probenabmessungen wurden zunächst anhand der Gleichung (1) die dehnungsabhängigen Spannungen errechnet. Für die Bewertung der mechanischen Eigenschaften wurde ausschließlich das Verhalten der bis zur Elastizitätsgrenze, also dem Erreichen der Spaltzugfestigkeit bzw. der Bruchdehnung betrachtet. Zur graphischen Darstellung der elastischen Eigenschaften der Xerogele wurden die für jede Probenzusammensetzung gemittelten Spaltzugfestigkeiten und zugehörigen Bruchdehnungen gegeneinander aufgetragen.

**Ausführungsbeispiel 7**

**Untersuchungen zur Biokompatibilität**

Mittels zellbiologischer und mikroskopischer Methoden sollten Aussagen zur Biokompatibilität der verwendeten Kollagene und hergestellten Silikat- und Hybrid-Xerogele getroffen werden.

**7.1 Sterilisation der hergestellten Proben**

Für den Einsatz in Zellkulturexperimenten wurden die hergestellten Proben (Kollagenbeschichtungen und Xerogele) von der Firma Gamma-Service Produktbestrahlung GmbH, Radeberg durch Gamma-Bestrahlung (Dosis: 25 kGy) sterilisiert.

**7.2 Verwendete Zellen**

Für die zellbiologischen Untersuchungen wurde die spontan immortalisierte Zelllinie 7F2 verwendet, die durch Isolation von Zellen aus dem Knochenmark des Oberschenkels (Femur) einer Maus und deren Klonierung gewonnen wurde [Thompson, D. L. ; Lum, K. D. ; Nygaard, S. C. ; Kuestner, R. E.; Kelly, K. A. ; Gimble, J. M. ; Moore, E. E.: The derivation and characterization of stromal cell lines from the bone marrow of p53-/- mice:new insights into osteoblast and adipocyte differentiation. In: J Bone Miner Res 13 (1998), Nr. 2, S. 195-204]. Diese osteoblastenähnlichen Zellen zeigen eine fibroblastenähnliche Gestalt und sind in vitro zu unbegrenztem Wachstum fähig. Die Kultivierung erfolgte bei 37°C und einem $CO_2$-Gehalt von 5 % mit Alpha-MEM8, dem zusätzlich 10 % FCS9, 100 U/ml P/S10 und 2 mM Glutamin zugegeben wurde.

### 7.3 Cytotoxizitätstest mittels LDH

Der Lactatdehydrogenase-Test wurde zur Bewertung der Cytotoxizität verschieden aufbereiteter Zustände des Chondrosia-Kollagens herangezogen [Takara Bio Inc: LDH Cytotoxicity Detection Kit Manual. v.03.01. - Cat.#MK401]. Als stabiles Enzym des Cytoplasmas wird die Lactatdehydrogenase (LDH) von nahezu allen Zelltypen synthetisiert. Unter der Voraussetzung konstanter Produktion kann die gemessene Enzymaktivität anhand einer Kalibrierreihe mit einer Zellzahl korreliert werden. Die verwendete Nachweismethode beruht auf einer enzymatischen Reaktion, bei der die LDH zunächst die Oxidation von Lactat zu Pyruvat katalysiert. Dabei wird NAD+ zu NADH/H+ reduziert. Anschließend werden die H/H+-Protonen des Katalysators (Diaphorase) auf das Tetrazoliumsalz INT (2-[4-lodophenyl]-3-[4-nitrophenyl]- 5-phenyltetrazolium) (gelb) übertragen, das daraufhin zu einem wasserlöslichen intensiv rot gefärbten Formazan reduziert wird. Die photometrisch gemessene Intensität des roten Farbstoffes ist direkt proportional zur LDH-Aktivität. Für den LDH-Test wurden jeweils $2 \times 104$ Zellen (Mausosteoblasten 7F2) in die Vertiefungen von 48er Wellplatten ausgesät und 12 Stunden kultiviert, so dass die Zellkultur zu Beginn des Tests noch nicht konfluent war. Zur Herstellung der Testlösungen wurden auf verschiedene Weise erhaltene Chondrosia-Kollagensuspensionen und Referenzlösungen im Verhältnis 1:10 (60 $\mu$l Suspension auf 540 $\mu$l Medium) in Zellkulturmedium gelöst, mit dem die Zellen anschließend kultiviert wurden. Diese Suspensionen waren:

1. Chondrosia-Kollagen lyophilisiert und gelöst in 0,1 M Tris/HCl pH 7,4 (0,1, 1,0, 10,0 mg/ml)
2. Überstände der in Punkt 1 genannten Suspensionen
3. Chondrosia-Kollagen wie in Punkt 1 nach thermischer Denaturierung (eine Stunde bei 95°C)
4. Überstände der in Punkt 3 genannten Suspensionen
5. Chondrosia-Kollagen nachWasserstoffperoxid-Behandlung, davon 10,0 mg/ml gelöst in 0,1 M Tris/HCl pH 7,4
6. Background (0,1 M Tris/HCl-Puffer pH 7,4 ohne Zellen)
7. Low Control (LC, 0,1 M Tris/HCl-Puffer pH 7,4)
8. High Control (HC, 20% Triton X-100)

Die Inkubation der Zellen wurde begonnen, indem bei drei Proben pro Zustand das reine Kulturmedium durch die oben genannten Kulturmedium-Suspensions-Mischungen ausgetauscht wurde. Bei Weiterkultivierung im Brutschrank wurden nach 1-, 4- und 24-stündiger Inkubation für den Test 50 $\mu$l des Zellkulturmediums von den jeweiligen Proben abgenommen, in Mikrotiterplatten pipettiert und anschließend zügig mit je 50 $\mu$l der im LDH-Kit enthaltenen Substratlösung versetzt. Die 20-minütige Inkubation wurde lichtgeschützt bei Raumtemperatur und unter leichtem Schütteln durchgeführt. Anschließend wurde die Reaktion durch Zugabe von 50 $\mu$l 0,5 N HCl abgestoppt und die Absorption des entstandenen Farbstoffes bei 492 nm gemessen. Durch Vierfach-Bestimmung wurden dabei Pipettier- und Verdünnungsfehler verringert. Nach Abzug des mittleren Absorptionswertes des Backgrounds wurde anhand der Referenz- und Probenwerte die Cytozoxizität jeder Probenlösung nach folgender Formel nach Takara Bio Inc: LDH Cytotoxicity Detection Kit Manual. v.03.01. - Cat.#MK401 berechnet.

### 7.4 Zellvitalitätstests mittels MTT

Der MTT-Test diente zur Bestimmung der Zellvitalität bzw. -proliferation bei Kultivierung der Mausosteoblasten auf den mit Chondrosia- und Kalbshautkollagen Typ I beschichteten, sowie als Referenz unbeschichteten Wellplatten [Berridge, M. V. ; Tan, A. S. ; McCoy, K. D. ; Wang, R.: The Biochemical and Cellular Basis of Cell Proliferation Assays That Use Tetrazolium Salts. In: Biochemica (1996), Nr. 4, S. 14-9]. Des Weiteren wurde die Vitalität der auf den Hybrid-Xerogelen (mit Chondrosia- und Kalbshautkollagen Typ I) kultivierten Zellen bestimmt. Dieser Test basiert auf der Umwandlung des schwach gelben 3-(4,5-dimethylthiazol-2-yl)-2,5-diphenyl Tetrazolium Bromids (MTT) durch Dehydrogenasen aktiver Mitochondrien zu dunkelblauem, wasserunlöslichem Formazan [Sigma Aldrich: MTT (3-[4,5-Dimethylthiazol-2-yl]-2,5-diphenyltetrazoliumbromide; Thiazolyl blue) Produktinformation. - Product No. M 5655]. Diese Umwandlung kann nur in lebenden, stoffwechselaktiven Zellen stattfinden. Das gebildete Formazan kann durch saures Isopropanol solubilisiert und die erhaltene Lösung anschließend photometrisch gemessen werden. Die Intensität des blauen Farbstoffes ist dabei direkt proportional zur Aktivität der Dehydrogenasen und somit auch zum Anteil der lebenden, stoffwechselaktiven Zellen.

Die verwendeten Xerogele wurden vor der Besiedlung über 24 Stunden in Zellkulturmedium eingelegt, um eine vollständige Benetzung und Tränkung zu erreichen. Anschließend wurden jeweils $6 \times 10^4$ Zellen (Mausosteoblasten 7F2) in die Vertiefungen der unbeschichteten und beschichteten (Chondrosia- und Kalbshautkollagen Typ I) Wellplatten bzw. im kleinen Volumen als Tropfen auf die Xerogele pipettiert. Nach 30-minütiger Adhäsionszeit wurde mit Zellkulturmedium aufgefüllt. Der MTT-Test wurde jeweils nach Ablauf der definierten Kultivierungszeiten (1, 3, 7 Tage) auf den entsprechenden Proben durchgeführt, um Rückschlüsse auf die Zellvitalität bzw. die entsprechende Zellzahl ziehen zu können. Zur Erstellung der Kalibrierreihen wurden bekannte Zellzahlen in Wellplatten ausgesät, 24 Stunden kultiviert und danach ebenfalls gemessen. Dabei wurde zunächst das Zellkulturmedium von den zu messenden Proben abgesaugt, wieder mit MTT-haltigem Medium (0,5 mg/ml) aufgefüllt und für vier Stunden im Brutschrank inkubiert. Nach Entfernung der MTT-Lösung wurde das Formazan durch Zugabe von 200 μl saurem Isopropanol (0,05 M HCl in Isopropanol) gelöst. Nach weiteren 15 Minuten Inkubation im Brutschrank wurden von jeder Probe 100 μl in Mikrotiterplatten umpipettiert und die Absorption des Farbstoffes mit dem Fluoreszenzplattenreader bei einer Wellenlänge von 570 nm (Referenzwellenlänge: 630 nm) gemessen. Als Leerwert wurde die Absorption von reinem Isopropanol bestimmt, die als Untergrund von allen anderen Absorptionswerten abgezogen wurde. Durch Korrelation der Absorptionswerte der Proben mit denen der Kalibrierreihen wurde auf die entsprechenden Zellvitalitäten bzw. -zahlen geschlossen.

**7.5 Fluoreszenz- und konfokale Laser-Scanning-Mikroskopie (FM, LSM)**

Fluoreszenz im sichtbaren Bereich ist das durch Absorption von Licht angeregte Leuchten eines Stoffes. Die Prozesse, die von der Absorption von Anregungslicht zur Emission von Fluoreszenzlicht führen, werden meist anhand der Energien charakteristischer Elektronenübergänge in so genannten Jablonski-Diagrammen dargestellt. Viele Fluoreszenzfarbstoffe (Fluorchrome) haben aromatische Ringstrukturen und besitzen delokalisierte Elektronen so genannten bindende ⊢-Orbitalen. Die Absorption eines Anregungsphotons kann ein Elektron aus dem Grundzustand in einen angeregten (energiereicheren) Zustand heben. Beim Übergang dieses Elektrons zurück in den Grundzustand wird die freiwerdende Energie als Fluoreszenzphoton emittiert. Da diese Prozesse immer mit Energieumwandlungen (Reorganisation der Lösungsmittelmoleküle, Wärmeentwicklung) verbunden sind, liegt die Energie des emittierten Photons immer unter der des absorbierten Photons. Damit ist die Wellenlänge des Fluoreszenzlichts größer als die des Anregungslichts (Stokes'sche Regel). Voraussetzung für die Fluoreszenzmikroskopie ist das Vorhandensein von Substanzen, die sich zur Fluoreszenz anregen lassen. Es gibt Substanzen (z. B. Chlorophyll in Blättern, Öle, Wachse) die natürlicherweise fluoreszieren, was als Primär- oder Autofluoreszenz bezeichnet wird. Oft zeigen auch Zellen und Gewebe eine solche, meist unerwünschte, unspezifische Eigen- oder Autofluoreszenz, die sich im Allgemeinen als weiß-blaues Leuchten des gesamten Präparats bei UV- und Violettanregung zeigt. Um jedoch bestimmte Objektstrukturen hervorheben, werden Fluorchrome induziert, die spezifisch an die interessierenden Strukturen koppeln. Die so erhaltene Fluoreszenz nennt man Sekundärfluoreszenz. Bei der konventionellen Fluoreszenzmikroskopie wird in der Regel mit Quecksilber-Höchstdrucklampen als Lichtquelle gearbeitet. Um die Fluoreszenzanregung aber mit bestimmtenWellenlängen vornehmen zu können, werden spezielle Anregungsfilter eingesetzt. Bei der Auflichtanregung (Epifluoreszenz), wie sie üblicherweise auch im Rahmen der vorliegenden Arbeit angewendet wurde, wird das Anregungslicht durch das gleichzeitig als Kondensor dienende Mikroskopobjektiv auf das Präparat gebracht. Das Licht wird dabei mithilfe eines dichromatischen Teilerspiegels, der für eine Trennung zwischen Anregungsund Fluoreszenzstrahlung sorgt, in den Strahlengang und ungeschwächt auf das Präparat gelenkt. Dagegen durchdringt die nach der Stokes'schen Regel längerwellige Fluoreszenzstrahlung des Präparats den Farbteiler und wird über das Okular sichtbar oder von einer Digitalkamera aufgenommen. Bei der konfokalen Laser-Scanning-Mikroskopie werden Laser definierter Wellenlänge als Strahlungsquelle verwendet und mithilfe eines Kollimators zu einem parallelen Strahlenbündel geformt, bevor sie auf die Probe treffen. Nach Passieren des Farbteilers wird das Fluoreszenzlicht ortsabhängig mit einem Detektor erfasst. Das auch in dieser Arbeit genutzte konfokale Prinzip ermöglicht es durch eine vor dem Detektor befindliche Blende (Pinhole), die Bildinformationen gezielt aus nur einer Ebene der dreidimensionalen Probe zu sammeln. Galvanometrisch betriebene Spiegelscanner erlauben dem fokussierten Laserstrahl ein punktuelles Abrastern in zwei Raumrichtungen (x, y), die z-Richtung wird durch die Bewegung des Objektivs gescannt. Auf diese Weise lassen sich gezielt optische Schnitte des Präparats aufnehmen, die später zu einem dreidimensionalen Bildstapel zusammengesetzt werden können.

**Fluoreszenzmarkierung der Zellen**

Für die Fluoreszenzfärbung wurden die Zellen durch Zugabe einer 3,7 %igen Formaldehyd/ PBS-Lösung für 10 Minuten bei 4°C fixiert. Dies geschieht durch die Quervernetzung entsprechender Proteine unter Beteiligung der Aldehydgruppen des Formaldehyds, wobei die Zellstruktur erhalten bleibt. Im selben Schritt wurde durch die Zugabe von 0,2% Triton X-100 eine Permeabilisierung der Zellmembranen und somit die Zugänglichkeit für die Fluoreszenzfarbstoffe erreicht. Anschließend wurden die Zellen dreimal für je fünf Minuten mit PBS gewaschen. Zur Lokalisierung und Beurteilung der Adhäsion mit dem Fluoreszenzmikroskop bzw. dem LSM wurde das Aktingerüst der Zellen mit dem grün fluoreszierenden Farbstoff Alexa Fluor 488-Phalloidin und die Zellkerne mit dem blau fluoreszierenden 4',6-Diamidino-2-phenylindoldihydrochlorid (DAPI) markiert [Molecular ProbesTM: Phallotoxins Produktinformation. 2006 - MP 00354, Sigma Aldrich: DAPI (4',6-Diamidino-2-phenylindoldihydrochlorid) Produktinformation 2005. - Product No. D 9542]. Die Farbstoffe wurden 1:100 (Alexa Fluor 488-Phalloidin) bzw. 1:1000 (DAPI) in PBS verdünnt und die Zellen für je eine Stunde bei Raumtemperatur im Dunkeln inkubiert. Danach wurden die Zellen dreimal mit PBS gespült und mit entionisiertem Wasser eingedeckt. Bis zur Mikroskopie wurden die Proben bei 4°C im Dunkeln aufbewahrt.

**Mikroskopie der Zellen**

Die Aufnahmen mit dem Fluoreszenz- und Laser-Scanning-Mikroskop wurden von Frau C. Knieb bzw. Herrn T. Hanke angefertigt. Das verwendete Laser-Scanning-Modul ist an ein Fluoreszenzmikroskop Axioskop 2 FS mot gekoppelt. Das Modul steuert einen Argonionenlaser, Helium-Neon-Laser und NIR-femtosekunden Titan-Saphir-Laser für die 2-Photonenanregung. Die Steuerung des Mikroskops und die primäre Bildverarbeitung erfolgte mittels geeigneter Software von Zeiss. Für die Aufnahmen wurden die Objektive Plan-Neofluar 10×/0.30, Plan-Neofluar 20×/0.50 und Plan-Apochromat 20×/0.75 verwendet. Die fluoreszenzmikroskopischen Aufnahmen wurden mit einer Digitalkamera Axiocam angefertigt und mit der Software AxioVision (Version 3.1, Zeiss Vision) verarbeitet, in der auch die Überlagerung von Einzelbildern zu Mehrkanalaufnahmen erfolgte. Die Verarbeitung von einzelnen LSM-Bildern und deren 3D-Rekonstruktion erfolgte mit der LSM 510 Geräte-Software.

**Ausführungsbeispiel 8:**

**Hybridmaterial aus Kalbshautkollagen und Silikat**

Rinderhäute wurden von einem Schlachthaus bezogen, in kaltem Wasser gewaschen, aufgespalten, gekalkt, gewaschen, entkalkt, mit Wasserstoffperoxid behandelt und grob in einem Fleischwolf gemahlen. Der pH-Wert des erhaltenen Materials wurde auf pH 3,5 eingestellt, mit Eis gemischt und in einer Kolloidmühle zerkleinert. Die erhaltene Kollagenpaste wurde in einer Christ Alpha 1-4 Gefriertrocknungsanlage lyophilisiert. Homogene Suspensionen wurden durch intensives Vermischen von 25mg Trockenmaterial pro Milliliter in 0,01 M HCl für 48h bei 4°C erhalten. Tetraethoxysilan (TEOS 99%, Roth) diente als Silikatprecursor, indem es 24h bei 4°C unter Zugabe von Wasser und HCl als Katalysator hydrolisiert wurde. Das molare Verhältnis von TEOS zu Wasser wurde auf 1:4 gewählt, was eine vollständige Hydrolyse des Silikat-Precursors ermöglicht, und das Mitführen überschüssigen Wassers vermeidet.

Die Hybridisierung - d. h. die Zusammenführung von Silikat und Kollagen - wurde durch intensive Vermischung des prehydrolisierten TEOS und der homogenen Kollagensuspension erreicht. Das erhaltene Masseverhältnis der Mischung entsprach 70% $SiO_2$ und 30% Kollagen. Nach 60 Sekunden wurde unter intensiver Vermischung ein entsprechendes Volumen 1 M Tris-HCl-Puffer (pH 9.0) zugegeben um insgesamt einen neutralen pH-Wert zu erhalten. Durch Evakuierung im Vakuum wurde das Einschließen von Luftblasen im Gel vermieden. Infolge der Polymerisation bildet sich das Hybridnetzwerk, die Viskosität steigt und der Gelpunkt wird nach ca. 10 Minuten erreicht. Das stabile Hydrogel kann bei Bedarf in einer steigenden Ethanolreihe entwässert werden. Da die Oberflächenspannung von Ethanol kleiner ist als die von Wasser, kann dadurch die bei der Trocknung auftretende Kapillarspannung reduziert werden, was sich günstig auf die physikalischen Eigenschaften des erhaltenen Xerogels auswirken kann. Unter Luftabschluss wurde vorzeitiges Antrocknen des Gels vermieden und im Rahmen der Alterung (Synerese) das Gelnetzwerk stabilisiert. Die Trocknung der Hydrogele - d. h. die Überführung in ein Xerogel - wurde unter verschiedenen klimatischen Bedingungen durchgeführt. Gemäß der DIN 50008 wurden gesättigte Salzlösungen in einem Exsikkator verwendet um eine relative Feuchte von 75% bei Temperaturen von 20°C, 40°C und 60°C einzustellen. Außerdem wurde das Trocknungsverhalten in Umgebungen von 20°C, 30% relative Feuchte und 4°C, 85% relative Feuchte untersucht. Die Evaluierung der verschiedenen Trocknungsparameter erfolgte über die zeitabhängige Bestimmung der Gelmassen in Intervallen von 24h. Ein Wert von 20% entspricht dabei einem vollständig getrockneten Xerogel.

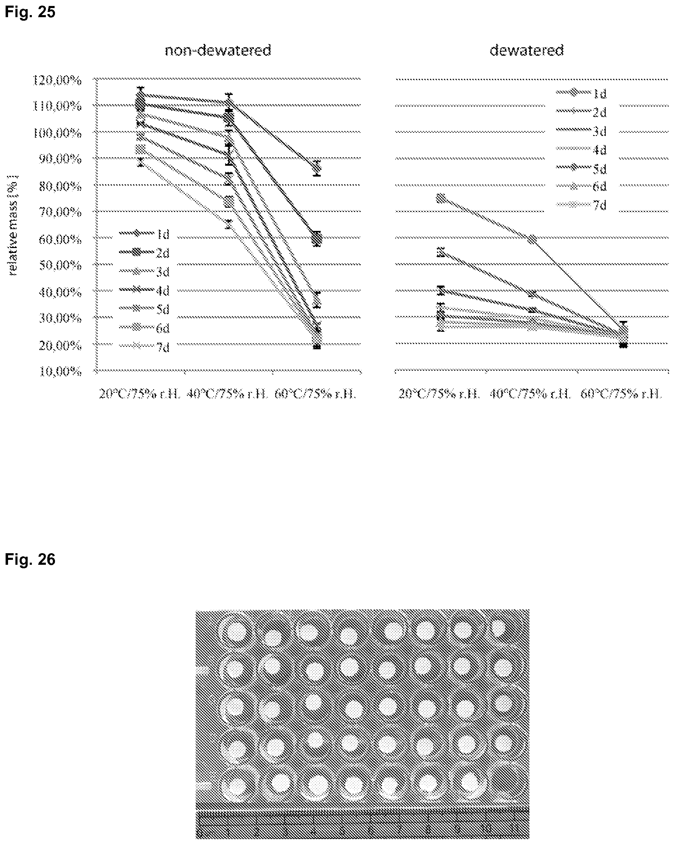

**Fig. 25** zeigt die relativen Gelmassen während des Trocknungsprozesses. Bei einer Temperatur von 20°C und 75% relative Feuchte nimmt die Masse der nicht entwässerten Proben innerhalb der ersten 7 Tage annähernd linear auf 88% ab. Im Gegensatz dazu nimmt die Masse des entwässerten Äquivalents im gleichen Zeitraum auf ca. 26% ab, was einer nahezu vollständigen Trocknung entspricht. Diese Proben weisen eine signifikante Schrumpfung auf, die aufgrund des Trocknungsgradienten zu einer leichten Deformierung - also Abweichung von der scheibenförmigen Zielgeometrie - führt. Bei etwa einem Viertel der Proben wurde die Festigkeit der Gele überschritten, was sich in makroskopisch sichtbarer Rissbildung auswirkte. Ähnliche Zustände, bei reduzierter Rissbildung (etwa 15% der Proben) zeigte sich bei der Trocknung bei 40°C und 75% relative Feuchte.
Bei einer Temperatur von 60°C bei ebenfalls 75% relativer Feuchte läuft die Trocknung der entwässerten sowie nicht entwässerten Proben deutlich beschleunigt ab. Beide Gruppen weisen einen asymptotisch fallenden Masseverlauf auf und erreichen ihre endgültige Xerogelmasse innerhalb von 7 Tagen. Alle Proben sind rissfrei und zeigen die scheibenförmige Zielgeometrie.

Zusammenfassend lässt sich feststellen, dass die Erhöhung der Temperatur bei einer relativen Luftfeuchte von 75% den Trocknungsprozess beschleunigt, das Auftreten von Trocknungsrissen minimiert und die Maßhaltigkeit der Probengeometrien unterstützt. Eine zuvor durchgeführte Entwässerung der Proben unterstützt diese positiven Effekte ebenfalls. Die unter optimierten Bedingungen (Entwässerung, Alterung, Trocknung bei 60°C, 75% relative Feuchte) erhaltenen Hybrid-Xerogele weisen typischerweise eine scheibenartige, sehr gut reproduzierbare Geometrie auf (siehe **Figur 26**).

Die Trocknung bei 4°C, 85% relative Feuchte bzw. 20°C, 30% relative Feuchte führte in den durchgeführten Experimenten zu stark verlangsamter Trocknung bzw. deutlicher Rissbildung in den Gelen und wurde daher als weniger geeignet eingestuft.

Die Biokompatibilität der Silikat-Kollagen-Hybridmaterialien (SiKoll) wurde durch die Kultivierung von humanen mesenchymalen Stammzellen auf den SiKoll-Xerogelen beurteilt. Die äquivalente Kultivierung auf Polystyrol diente zur Kontrolle. Für die Zellkultivierung wurde in 37°C bei 7% $CO_2$ Alpha-MEM (minimal essential medium) mit zusätzlich 10% fetales Kälberserum und 1% Penicillin/Streptomycin verwendet. Das Medium wurde alle zwei Tage erneuert. Drei Tage nach der Aussaat wurde die osteogene Differenzierung durch die Zugabe von 10nM Dexamethason, 10nM 1,25dihydroxy-Vitamin D3 und 3,5mM beta-Glycerophosphat in das Medium eingeleitet.

Biochemische Untersuchungen:

Für biochemische Analysen wurden am Tag 1, 3, 7, 14 und 21 je drei Proben untersucht. Zur Analyse wurden die Zellen mit 1 % Triton X-100 lysiert. Die Zellproliferation wurde durch Bestimmung des DNA-Gehalts beurteilt. Die Bestimmung der Aktivität der alkalischen Phosphatase diente zur Beurteilung der osteogenen Differenzierung der Zellen. Für die fluoreszenzmikroskopischen Untersuchungen wurden die Zellen auf den Xerogelproben mit PBS gewaschen, fixiert, permeabilisiert und mit 1% BSA (bovine serum albumin) geblockt. Das Zytoskellet (Actin) der Zellen wurde mit dem Farbstoff AlexaFluor 488, die Zellkerne mit dem Farbstoff 4',6-diamidino-2phenylindole markiert. ALP nach 14 Tagen wurde mittels dem ELF®97 Endogenous Phosphatase Detection Kit markiert. Die Fluoreszenz- und Laser-Scanning-Mikroskopie wurde wie bereits beschrieben durchgeführt.

Exakt dieselben Proben, die zur Fluoreszenzmikroskopie verwendet wurden, wurden in einer steigenden Ethanolreihe entwässert, kritisch-punkt-getrocknet und mit Gold bedampft. Die Silikatisierung der Fibrillen sowie die hierarchische Struktur des Hybridmaterials konnte auf diese Weise mittels Rasterelektronenmikroskopie visualisiert werden.

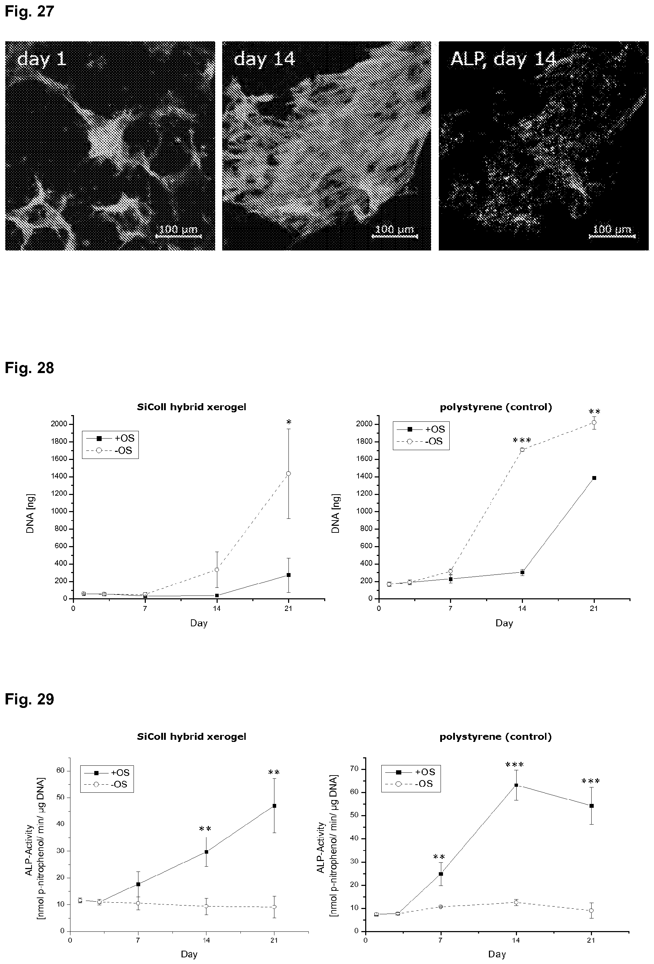

Die Laser-Scanning-Mikroskopie zeigte, dass die verwendeten Zellen sehr gut auf dem Silikat-Kollagen-Hybridxerogel adherieren (**Fig. 27**). Nach 14-tägiger Kultivierungsdauer ist eine dichte Zellschicht festzustellen, was eine gute Proliferation der Zellen auf dem Material verdeutlicht (**Fig. 27**). Außerdem lässt sich nach dieser Zeitperiode eine starke Fluoreszenz der ELF®97-Substanz feststellen, was auf die gewünschte Differenzierung der hMSCs in osteoblastenartige Zellen hinweist (rechtes Teilbild in **Fig. 27**).

Qualitative Messungen bezüglich Zellproliferation und -differenzierung wurden über einen Zeitraum von 21 Tagen durchgeführt. Es wurden dabei osteogen induzierte Zellen (+OS) mit nicht-induzierten Zellen (-OS) verglichen. Als Kontrolle dienten die gleichen Bedingungen bei Kultivierung auf Polystyrol. Die Analyse des DNA-Gehalts zeigte eine deutliche Zunahme der Zellzahl (**Fig. 28**).

Bei der Zellkultivierung auf den Silikat-Kollagen-Hybridxerogelen sowie bei den Kontrollproben ist bei nicht erfolgter Induzierung erwartungsgemäß kein Anstieg der ALP-Aktivität festzustellen **(Fig. 29).** Durch die Zugabe der Differenzierungshilfsmittel ist bei allen Proben ein steter Anstieg der auf den DNA-Gehalt bezogenen ALP-Aktivität festzustellen, was die beabsichtigte Differenzierung in osteogene Richtung belegt. Das Erreichen eines Maximums ist mit einsetzender Matrixmineralisierung und der anschließenden Differenzierung in Osteocyten zu erklären. Die gemeinsame Betrachtung der zeitlichen Zellproliferation und ALP-Aktivität legt nahe, dass die osteogen induzierten Zellen vorrangig differenzieren auf Kosten verringerter Proliferation.

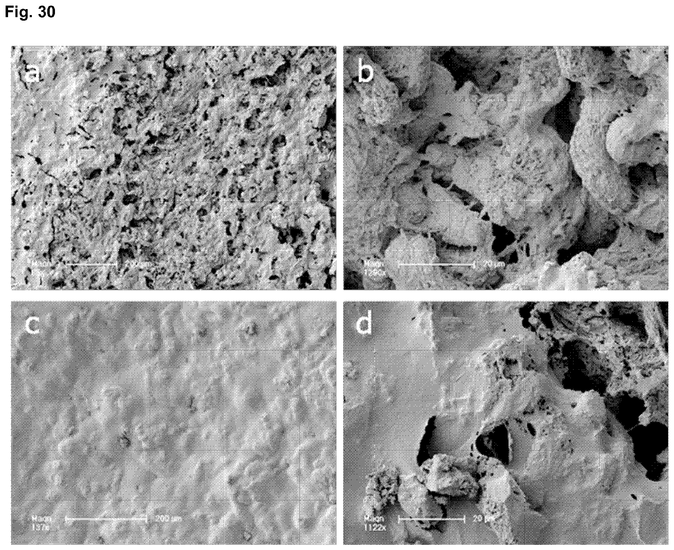

Rasterelektronenmikroskopie wurde eingesetzt, um die Morphologie der Hybridxerogele sowie die Zelladhäsion und -proliferation zu beurteilen. Die Bilder in **Fig. 30** repräsentieren die aus 70% Silikat und 30% Kollagen bestehenden Xerogele. Es ist zu erkennen wie durch gleichmäßige Silikatisierung der Kollagenfibrillen ein Hybridmaterial entstanden ist. Außerdem zeigt sich wie zuvor festgestellt, dass sich die Zellen sehr gut an den Untergrund anpassen können und zu einer dichten Zellschicht proliferieren.

## Patentansprüche

1. Hybridmaterial bestehend aus einer silikatisierten Kollagenmatrix, wobei das Kollagen rekombinantes Kollagen, Kollagen aus Eumetazoa, Schwammkollagen der Klassen Demospongia (Hornschwämme) oder Calcarea (Kalkschwämme), ein synthetisches Kollagenanalogon, ein Kollagenderivat oder eine Mischung dieser Kollagene ist und wobei die Kollagenmatrix durch die Silikatisierung der Kollagenfibrillen versteift und vernetzt ist und der Anteil an Kollagen im Hybridmaterial 30 bis 70 Masse-% beträgt.

2. Silikatisiertes poröses Kollagenscaffold, erhältlich durch Gefriertrocknung eines Hybridmaterials nach Anspruch 1.

3. Xerogel aus einem Hybridmaterial nach Anspruch 1, erhältlich dadurch, dass eine homogene Kollagensuspension und eine hydrolysierte Alkoxysilan-Lösung gemischt werden, das Produkt nach Erreichen des Gelpunkts zur Festigung der Festkörperstruktur in einem geeigneten Lösungsmittel gelagert und das so erhaltene Hydrogel anschließend getrocknet wird.

4. Aerogel aus einem Hybridmaterial nach Anspruch 1, erhältlich dadurch, dass eine homogene Kollagensuspension und eine hydrolysierte Alkoxysilan-Lösung gemischt werden, das Produkt nach Erreichen des Gelpunkts zur Festigung der Festkörperstruktur in einem geeigneten Lösungsmittel unter Luftabschluss gelagert, die Flüssigphase des so erhaltenen Hydrogels substituiert wird und im Anschluss eine Trocknung unter überkritischen Bedingungen durchgeführt wird.

5. Partikel aus einem Hybridmaterial nach Anspruch 1.

6. Beschichtungsmaterial aus einem Hybridmaterial nach Anspruch 1.

7. Verfahren zur Herstellung eines Hybridmaterials aus einer silikatisierten Kollagenmatrix, wobei als Kollagen rekombinantes Kollagen, Kollagen aus Eumetazoa, Schwammkollagen aus einem Schwamm der Klasse Demospongia (Hornschwämme) oder Calcarea (Kalkschwämme), ein synthetisches Kollagenanalogon, ein Kollagenderivat oder eine Mischung dieser Kollagene verwendet wird, wobei die Kollagenmatrix durch die Silikatisierung der Kollagenfibrillen versteift und vernetzt wird und der Anteil an Kollagen im Hybridmaterial 30 bis 70 Masse-% beträgt, indem eine homogene Kollagensuspension und eine hydrolysierte Alkoxysilan-Lösung oder Wasserglas ($Na_2SiO_3$ als Silizium-Precursor unter Rühren vermischt werden.

8. Verfahren nach Anspruch 7, **dadurch gekennzeichnet, dass** als Alkoxysilan-Lösung eine Tetramethoxysilan (TMOS)-Lösung oder eine Tetraethoxysilan (TEOS)-Lösung eingesetzt wird.

9. Verwendung eines Hybridmaterials nach Anspruch 1 als Konstruktionswerkstoff und/oder als Funktionswerkstoff oder als Implantatmaterial oder in elektronischen Bauelementen.

10. In vitro Verwendung eines Scaffolds nach Anspruch 2 zum Tissue Engineering.

11. Xerogel oder Aerogel nach Anspruch 3 oder 4 zur Verwendung als Zahnfüllung, als Implantatmaterial, als Knochenersatzmaterial, für die Fixierung von Knochenbrüchen oder für die Knochenregeneration.

12. Verwendung eines Partikels nach Anspruch 5 als Wirkstoffträger.

13. Verwendung einer Beschichtung nach Anspruch 6 zur Biologisierung von Implantatoberflächen.

14. Knochenersatzmaterial enthaltend ein Hybridmaterial nach einem der Ansprüche 1 bis 4.

**Claims**

1. Hybrid material consisting of a silicatized collagen matrix, wherein the collagen is recombinant collagen, collagen from eumetazoa, sponge collagens of the classes Demospongia (horn sponges) or Calcarea (calcareous sponges), a synthetic collagen analog, a collagen derivative or a mixture of these collagens and wherein the collagen matrix is stiffened and cross-linked by the silicatization of the collagen fibrils and the proportion of collagen in the hybrid material is 30 to 70% by mass.

2. A silicatized porous collagen scaffold obtainable by freeze drying a hybrid material according to claim 1.

3. Xerogel of a hybrid material according to claim 1, obtainable by mixing a homogeneous collagen suspension and a hydrolyzed alkoxysilane solution, storing the product in a suitable solvent after reaching the gel point to consolidate the solid body structure, and subsequently drying the hydrogel thus obtained.

4. Aerogel of a hybrid material according to claim 1, obtainable by mixing a homogeneous collagen suspension and a hydrolyzed alkoxysilane solution, storing the product airtight in a suitable solvent after reaching the gel point to consolidate the solid body structure, substituting the liquid phase of the hydrogel thus obtained, and then performing a drying under supercritical conditions.

**5.** Particle from a hybrid material according to claim 1.

**6.** Coating material of a hybrid material according to claim 1.

**7.** Method for producing a hybrid material from a silicatized collagen matrix, wherein recombinant collagen, collagen from eumetazoa, sponge collagen from a sponge of the class Demospongia (horn sponges) or Calcarea (calcareous sponges), a synthetic collagen analogue, a collagen derivative or a mixture of these collagens is used as a collagen, wherein the collagen matrix is stiffened and cross-linked by the silicatization of the collagen fibrils, and the proportion of collagen in the hybrid material is 30 to 70% by mass, in which a homogeneous collagen suspension and a hydrolyzed alkoxysilane solution or waterglass ($Na_2SiO_3$) are mixed with stirring as a silicon precursor.

**8.** Method according to claim 7, **characterized in that** a tetramethoxysilane (TMOS) solution or a tetraethoxysilane (TEOS) solution is used as alkoxysilane solution.

**9.** Use of a hybrid material according to claim 1 as a construction material and/or as a functional material or as an implant material or in electronic components.

**10.** In vitro use of a scaffold according to claim 2 for tissue engineering.

**11.** Xerogel or aerogel according to claim 3 or 4 for use as a dental filling, as implant material, as bone replacement material, for the fixation of broken bones or for bone regeneration.

**12.** Use of a particle according to claim 5 as a drug carrier.

**13.** Use of a coating according to claim 6 for the biologizing of implant surfaces.

**14.** Bone replacement material containing a hybrid material according to one of claims 1 to 4.


**Revendications**

**1.** Matière hybride constituée d'une matrice de collagène silicatée, le collagène étant un collagène recombinant, un collagène eumétazoaire, un collagène d'éponge de la classe Démospongia (éponge en corne) ou Calcarea (éponge calcaire), un analogue synthétique de collagène, un dérivé de collagène ou un mélange desdits collagènes et la matrice de collagène étant rigidifiée et réticulée par silicatisation des fibrilles de collagène et la part de collagène dans la matière hybride s'élevant à de 30 à 70 % en masse.

**2.** Scaffold de collagène poreux silicaté, susceptible d'être obtenu par cryodessiccation d'une matière hybride selon la revendication 1.

**3.** Xérogel à partir d'une matière hybride selon la revendication 1, susceptible d'être obtenu en ce qu'on mélange une suspension de collagène homogène et une solution d'alcoxysilane hydrolysé, après l'atteinte du point de gélification, on la stocke dans un solvant adapté, pour solidifier la structure de corps solide et on fait sécher par la suite l'hydrogel ainsi obtenu.

**4.** Aérogel à partir d'une matière hybride selon la revendication 1, susceptible d'être obtenu en ce qu'on mélange une suspension de collagène homogène et une solution d'alcoxysilane hydrolysé, après l'atteinte du point de gélification, on stocke le produit sous vide dans un solvant adapté pour solidifier la structure de corps solide, on substitue la phase liquide de l'hydrogel ainsi obtenu et par la suite, on procède à un séchage sous de conditions supercritiques.

**5.** Particule d'une matière hybride selon la revendication 1.

**6.** Matière de revêtement à partir d'une matière hybride selon la revendication 1.

**7.** Procédé destiné à fabriquer une matière hybride à partir d'une matrice de collagène silicatée, en tant que collagène étant utilisé un collagène recombinant, un collagène eumétazoaire, un collagène d'éponge de la classe Démospongia (éponge en corne) ou Calcarea (éponge calcaire), un analogue synthétique de collagène, un dérivé de collagène ou un mélange desdits collagènes, la matrice de collagène étant rigidifiée et réticulée par silicatisation des fibrilles de collagène et la part de collagène dans la matière hybride s'élevant à de 30 à 70 % en masse, en ce qu'on mélange en brassant une suspension de collagène homogène et une solution d'alcoxysilane hydrolysé ou de verre soluble ($Na_2SiO_3$) en tant que précurseur de silicium.

8. Procédé selon la revendication 7, **caractérisé en ce qu'**en tant que solution d'alcoxysilane, on met en oeuvre une solution de tétraméthoxysilane (TMOS) ou une solution de tétraéthoxysilane (TEOS).

9. Utilisation d'une matière hybride selon la revendication 1 en tant que matière pour la construction et/ou en tant que matière fonctionnelle ou en tant que matière pour implant ou dans des composants électroniques.

10. Utilisation in vitro d'un scaffold selon la revendication 2 pour l'ingénierie tissulaire.

11. Xérogel ou aérogel selon la revendication 3 ou 4, destiné à l'utilisation en tant que matière de comblement dentaire, en tant que matière pour implant, en tant que matière pour substitut osseux, pour la fixation de fractures osseuses ou pour la régénération osseuse.

12. Utilisation d'une particule selon la revendication 5 en tant que support de principe actif.

13. Utilisation d'un revêtement selon la revendication 6, pour la biologisation de surfaces d'implants.

14. Matière pour substitut osseux contenant une matière hybride selon l'une quelconque des revendications 1 à 4.

**Fig. 1**

**Fig. 2**

**Fig. 3**

**Fig. 4**

**Fig. 5**

**Fig. 6**

**Fig. 7**

**Fig. 8**

**Fig. 9**

**Fig. 10**

**Fig. 11a**

| Linienfarbe Dichte [g/cm³] Prozentuales Masseverhältnis [SiO₂:Kollagen] | TMOS Konzentration [M] | | |
|---|---|---|---|
| | 1,00 | 0,75 | 0,50 |
| 0 | 1,573±0,039 100:0 | | |
| 25 | 1,223±0,048 70:30 | 1,305±0,028 64:36 | 1,461±0,026 54:46 |
| 50 | 1,339±0,025 54:46 | 1,410±0,051 47:53 | 1,412±0,038 37:63 |
| 75 | 1,331±0,061 44:56 | 1,300±0,064 37:63 | 1,380±0,078 28:72 |

**Fig. 11b**

**Fig. 12**

**Fig. 13**

**Fig. 14**

Fig. 15

Fig. 16

**Fig. 17**

**Fig. 18**

**Fig. 19**

**Fig. 20**

**Fig. 21**

**Fig. 22**

**Fig. 23**

**Fig. 24**

**Fig. 25**

**Fig. 26**

**Fig. 27**

**Fig. 28**

**Fig. 29**

**Fig. 30**

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0415183 A2
- EP 0450357 A2

- EP 1259120 A


**In der Beschreibung aufgeführte Nicht-Patentliteratur**

- **WINTERMANTEL, E.** Ha, S.-W.: Biokompatible Werkstoffe und Bauweisen. Springer Verlag, 1998
- **GELINSKY, M. ; KÖNIG, U. ; SEWING, A. ; POMPE, W.** Poröse Scaffolds aus mineralisiertem Kollagen - ein biomimetisches Knochenersatzmaterial. In. *Materialwiss Werkstofftech,* 2004, vol. 35 (4), 229-33
- **MIYAZAKI, T. ; OHTSUKI, C. ; TANIHARA, M.** Synthesis of bioactive organic-inorganic nanohybrid for bone repair through sol-gel processing. *In: J Nanosci Nanotechnol,* 2003, vol. 3 (6), 511-5, 1533-4880
- **NASSIF, N. ; BOUVET, O. ; NOELLE RAGER, M. ; ROUX, C. ; CORADIN, T.** Livage, J.: Living bacteria in silica gels. *In: Nat Mater,* 2002, vol. 1 (1), 42-4, 1476-1122
- **LIVAGE, J. ; CORADIN, T. ; ROUX, C.** Encapsulation of biomolecules in silica gels. *In: J Phys Condens Matter,* 2001, vol. 190, 673-91, 0953-8984
- **EHRLICH, H. ; HANKE, T. ; SIMON, P. ; GOEBEL, C. ; HEINEMANN, S. ; BORN, R. ; WORCH, H.** Demineralization of natural silica based biomaterials: new strategy for the isolation of organic frameworks. *In: BIOmaterialien,* 2005, vol. 6 (4), 297-302
- **EHRLICH, H. ; ERESKOVSKII, A. V. ; DROZDOV, A. L. ; KRYLOVA, D. D. ; HANKE, T. ; MEISSNER, H. ; HEINEMANN, S. ; WORCH, H.** A Modern Approach to Demineralization of Spicules in Glass Sponges (Porifera: Hexactinellida) fort he Purpose of Extraction and Examination of the Protein Matrix. *In: Russ Marin Biol,* 2006, vol. 32 (3), 186-93
- **LEVI, C. ; BARTON, J. L. ; GUILLEMET, C. ; LE BRAS, E. ; LEHUEDE, P.** A remarkably strong natural glassy rod: the anchoring spicule of the Monorhaphis sponge. *In: J Mater Sci Lett,* 1989, vol. 3, 337-9
- **ONO, Y. ; KANEKIYO, Y. ; INOUE, K. ; HOJO, J. ; NANGO, M. ; SHINKAI, S.** Preparation of Novel Hollow Fiber Silica Using Collagen Fibers as a Template. *In: Chem Lett,* 1999, 475-6

- **EGLIN, D. ; CORADIN, T. ; GIRAUD GUILLE, M. M. ; HELARY, C. ; LIVAGE, J.** Collagen-silica hybrid materials: sodium silicate and sodium chloride effects on type I collagen fibrillogenesis. *In: Biomed Mater Eng,* 2005, vol. 15 (1-2), 43-50, 0959-2989
- A novel route to collagen-silica biohybrids, in: Organic/Inorganic Hybrid Materials. **CORADIN et al.** Materials Research Society Symposium Proceedings. 2002, vol. 726, 79-83
- **EGLIN et al.** *Collagen-silica hybrid materials: Sodium silicate and sodium chloride effects on type I collagen fibrillogenesis, Bio-Medical Materials and Engineering,* 2005, vol. 15, 43-49
- **JUNGUA et al.** *Biochemical and morphological studies on the collagen of horny sponges. Ircinia filaments compared to spongines, Connective Tissue Research,* 1974, vol. 2, 193-203
- **SWATSCHEK et al.** Marine sponge collagen: isolation, characterization and effects on the skin parameters surface-pH, moisture and sebum. *European Journal of Pharmaceutics and Biopharmaceutics,* 2002, vol. 53, 107-113
- **SACHLOS, E. ; CZERNUSZKA, J. T.** Making tissue engineering scaffolds work. Review: the application of solid freeform fabrication technology to the production of tissue engineering scaffolds. *In: Eur Cell Mater,* 2003, vol. 5, 29-39
- **KORTESUO, P.** *Sol-gel derived silica gel monoliths and microparticles as carrier in controlled drug delivery in tissue administration,* 2001
- **THOMPSON, D. L. ; LUM, K. D. ; NYGAARD, S. C. ; KUESTNER, R. E. ; KELLY, K. A. ; GIMBLE, J. M. ; MOORE, E. E.** The derivation and characterization of stromal cell lines from the bone marrow of p53-/- mice:new insights into osteoblast and adipocyte differentiation. *In: J Bone Miner Res,* 1998, vol. 13 (2), 195-204
- **BERRIDGE, M. V. ; TAN, A. S. ; MCCOY, K. D. ; WANG, R.** The Biochemical and Cellular Basis of Cell Proliferation Assays That Use Tetrazolium Salts. *In: Biochemica,* 1996, (4), 14-9